# EUROPÄISCHE PATENTSCHRIFT

(11) **EP 1 054 869 B1**
(45) Veröffentlichungstag und Bekanntmachung des Hinweises auf die Patenterteilung: **22.09.2004**
(21) Anmeldenummer: 99910126.4
(22) Anmeldetag: 09.02.1999
(51) Int. Cl.: C07D 221/16, A61K 31/435, C07D 221/12, C07D 491/04, C07D 405/04

(54) **3,4-DIHYDROCHINOLIN-DERIVATIVE ALS STICKSTOFFMONOXID-SYNTHASE (NOS) INHIBITOREN**
3,4-DIHYDROQUINOLINE DERIVATIVES AS NITROGEN MONOXIDE SYNTHASE (NOS) INHIBITORS
DERIVES DE 3,4-DIHYDROQUILINE UTILISES COMME INHIBITEURS DE SYNTHASES-MONOXYDE D'AZOTE (NOS)

(30) Priorität: 12.02.1998 DE 19806348
(43) Veröffentlichungstag der Anmeldung: 29.11.2000
(73) Patentinhaber: SCHERING AKTIENGESELLSCHAFT, 13353 Berlin (DE)
(72) Erfinder: JAROCH, Stefan, D-10625 Berlin (DE); REHWINKEL, Hartmut, D-12051 Berlin (DE); HÖLSCHER, Peter, D-10559 Berlin (DE); SÜLZLE, Detlev, D-10589 Berlin (DE); HILLMANN, Margrit, D-13437 Berlin (DE); BURTON, Gerardine, Anne, D-13591 Berlin (DE); McDONALD, Fiona, Mcdougall, D-14055 Berlin (DE)
(74) Vertreter: Pohlman, Sandra M.
(86) Internationale Anmeldenummer: PCT/DE1999/000382
(87) Internationale Veröffentlichungsnummer: WO 1999/041240

(56) Entgegenhaltungen:
- WO-A-95/11231
- WO-A-96/14844
- WO-A-97/16430
- CHEMICAL ABSTRACTS, vol. 117, no. 9, 31. August 1992 Columbus, Ohio, US; abstract no. 90201c, NAGARAJAN, KUPPUSWAMY ET AL.: "Condensed heterotricyclics: synthesis of pyrazolo(3,4,-c)quinoline derivatives." XP002106699 & INDIAN J. CHEM., SECT. B, Bd. 31B(6), - 1992 Seiten 316-321, -& DATABASE CHEMICAL ABSTRACTS CA117:90201c, XP002106700

## Beschreibung

Die Erfindung betrifft 3,4-Dihydrochinolin-Derivate, ein Verfahren zu ihrer Herstellung und ihre Verwendung in Arzneimitteln.

In menschlichen Zellen existieren 3 spezifische Formen von Stickstoffmonoxid-Synthasen, die Arginin in Stickstoffmonoxid (NO) und Citrullin überführen. So wurden zwei konstitutive NO-Synthasen (NOS) identifiziert, die als Ca⁺⁺/Calmodulin abhängige Enzyme im Gehirn (ncNOS oder NOS 1) bzw. im Endothel (ecNOS oder NOS 3) vorhanden sind. Die dritte Isoform ist die induzierbare NOS (iNOS oder NOS 2), die ein Ca⁺⁺ unabhängiges Enzym ist und nach Aktivierung unterschiedlicher Zellen durch Endotoxin oder andere Stoffe induziert wird.

NOS-Inhibitoren und insbesondere spezifische Inhibitoren der NOS 1, NOS 2 oder NOS 3 sind daher zur Therapie unterschiedlicher Erkrankungen geeignet, die durch pathologische Konzentrationen von NO in Zellen hervorgerufen oder verschlimmert werden (Clin. Neuropharmac. 18, 1995 Seite 482).

Die Synthese von Pyrazolo[3,4-c]-chinolin-Derivaten wie 2H,2-Methylpyrazolo[3,4-c]-chinolin-4-N,N-diethylamin ist aus Chem. Abs. 117: 90201 c bekannt. Cyclische Amidin-Analoge mit NOS-inhibitorischer Aktivität werden in WO 96/14844 beschrieben.

Es wurde nun gefunden, daß die erfindungsgemäß substituierten Heterocyclen besonders vorteilhaft als Arzneimittel eingesetzt werden können.

Die Erfindung betrifft die Verbindungen der Formel I, deren tautomere und isomere Formen und deren physiologisch verträgliche Salze 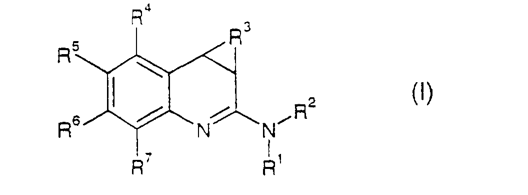 worin
R¹ und R² unabhängig voneinander bedeuten
a) Wasserstoff,
b) C₁₋₆-Alkyl,
c) OR⁸,
d) NR⁸R⁹,
e) CN,
f) Acyl,
g) CO₂R¹⁰,
h) CONR⁸R⁹,
i) CSNR⁸R⁹,
R³ bedeutet
einen gesättigten oder ungesättigten C₁₋₅-Alkylenrest, der 1- bis 4-fach mit OR¹¹, NR¹²R¹³ oder C₁₋₄-Alkyl substituiert sein kann und bei dem 1 oder 2 CH₂-Gruppen durch O, S(O)ₙ, NR¹⁴, =N- oder Carbonyl ersetzt sein können und der mit einer Methano-, Ethano- oder Propano-Gruppe über brückt sein kann,
R⁴, R⁵, R⁶ und R⁷ unabhängig voneinander bedeuten
a) Wasserstoff,
b) Halogen,
c) S(O)ₙR¹⁶,
d) OR¹⁶,
e) COOR¹⁶,
f) COR¹⁶,
g) CONR¹⁶R¹⁷,
h) CSNR¹⁶R¹⁷,
i) C(NR¹⁸)NR¹⁶R¹⁷,
j) NR¹⁶R¹⁹,
k) C₁₋₆-Alkyl, das gegebenenfalls substituiert ist mit Halogen, OR¹⁶, SR¹⁶, COOR¹⁶, Phenyl, 5-6-gliedrigem Heteroaryl mit 1-4 N-, S- oder O-Atomen oder C₃₋₇-Cycloalkyl,
l) C₃₋₇-Cycloalkyl,
m) C₂₋₆-Alkenyl, gegebenenfalls substituiert mit Phenyl oder Halogen,
n) C₂₋₆-Alkinyl, gegebenenfalls substituiert mit Phenyl oder Halogen,
o) C₆₋₁₀-Aryl, das gegebenenfalls substituiert ist mit Halogen, CN, C₁₋₄-Alkyl, SR¹⁶ oder OR¹⁶,
p) 5-6-gliedriges Hetaryl mit 1 bis 4 N-, O- oder S-Atomen, das einen ankondensierten Benzolring enhalten und substituiert sein kann mit Halogen, NO₂, Cyano, -OR¹⁶, SR¹⁶, C₁₋₄-Alkyl, CF₃ oder NR¹⁶R¹⁷
q) CN,
r) NO₂,
s) CF₃,
t) OCF₃,
R⁸, R⁹, R¹⁰, R¹¹, R¹⁶, R¹⁸, R²¹ und R²² unabhängig voneinander bedeuten
a) Wasserstoff,
b) C₁₋₆-Alkyl,
c) C₆₋₁₀-Aryl, das gegebenenfalls substituiert ist mit Halogen oder C₁₋₄-Alkyl
R²⁰ bedeutet
a)C₁₋₆-Alkyl,
b) C₆₋₁₀-Aryl bedeutet, das gegebenenfalls substituiert ist mit Halogen oder C₁₋₄-Alkyl,
R¹², R¹³, R¹⁴ und R¹⁹ unabhängig voneinander bedeuten
a) Wasserstoff,
b) C₁₋₆-Alkyl,
b) COR²⁰,
c) CO₂R²¹,
d) CONR²¹R²²,
e) CSNR²¹ R²²,
R¹⁷ bedeutet
a) Wasserstoff,
b) C₁₋₆-Alkyl, gegebenenfalls substituiert mit Halogenen, Amino-, Hydroxy- oder Sulfhydrylgruppen,
c) C₆₋₁₀-Aryl,
n bedeutet 0,1,2 und
R¹⁶, R¹⁹ gemeinsam mit dem Stickstoffatom einen gesättigten 5-, 6- oder 7-gliedrigen Ring bilden, der ein weiteres Stickstoff-, Sauerstoff- oder Schwefelatom enthalten und substituiert sein kann mit C₁₋₄Alkyl oder einem gegebenenfalls mit Halogen substituierten Phenyl-, Benzyl- oder Benzoyl-Rest.

Die Verbindungen der Formel können als Tautomere, Stereoisomere oder geometrische Isomere vorliegen. Die Erfindung umfaßt auch alle möglichen Isomere wie E- und Z-Isomere, S- und R-Enantiomere, cis- und trans-Diastereomere, Racemate und Gemische derselben einschließlich der tautomeren Verbindungen der Formel Ia und Ib (für R² = Wasserstoff).

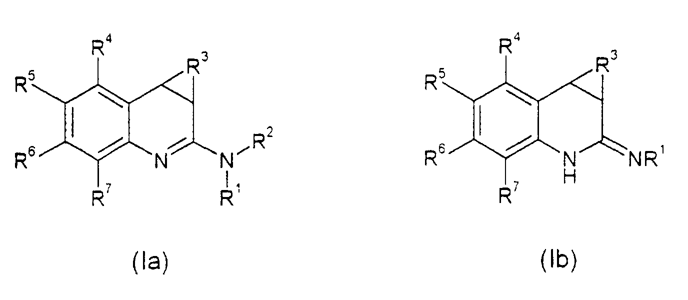

Die physiologisch verträglichen Salze können mit anorganischen und organischen Säuren gebildet werden wie beispielsweise Oxalsäure, Milchsäure, Zitronensäure, Fumarsäure, Essigsäure, Maleinsäure, Weinsäure, Phosphorsäure, Salzsäure, Bromwasserstoffsäure, Schwefelsäure, p-Toluolsulfonsäure, Methansulfonsäure u.a.

Zur Salzbildung von Säuregruppen sind auch die anorganischen oder organischen Basen geeignet, die zur Bildung physiologisch verträglicher Salze bekannt sind wie beispielsweise Alkalihydroxide, wie Natrium- und Kaliumhydroxid, Erdalkalihydroxide wie Calciumhydroxid, Ammoniak, Amine wie Ethanolamin, Diethanolamin, Triethanolamin, N-Methylglucamin, Tris-(hydroxymethyl)-methylamin usw.

Alkyl bedeutet jeweils eine geradkettige oder verzweigte Alkylgruppe wie z.B. Methyl, Ethyl, Propyl, Isopropyl, n-Butyl, sek-Butyl, tert-Butyl, n-Pentyl, sek-Pentyl, tert-Pentyl, Neopentyl, n-Hexyl, sek-Hexyl, Heptyl, Octyl, wobei 1 bis 4 Kohlenstoffatome bevorzugt sind.

Ist der Alkylrest halogeniert, so kann dieser ein- oder mehrfach halogeniert vorliegen, wobei Trifluormethyl bevorzugt ist.

Alkenyl- und Alkinyl-Reste sind jeweils geradkettig oder verzweigt und enthalten bevorzugt bis zu 4 Kohlenstoffatome. Beispielsweise seien die folgenden Reste genannt: Vinyl, 2-Propenyl, 1-Propenyl, 2-Butenyl, 1-Butenyl, 1-Butenyl, 2-Butenyl, 1-Methyl-1-propenyl, 2-Methyl-2-propenyl, 3-Methyl-2-propenyl, Ethinyl, 1-Propinyl, 2-Propinyl, 1-Butinyl, 2-Butinyl.

Unter Cycloalkyl ist jeweils Cyclopropyl, Cyclobutyl, Cyclopentyl, Cyclohexyl oder Cycloheptyl zu verstehen.

Halogen bedeutet jeweils Fluor, Chlor, Brom oder Iod.

Unter Aryl ist jeweils Naphthyl oder insbesondere Phenyl zu verstehen, die einoder dreifach, gleich oder verschieden substituiert sein können.

Die Hetarylreste können einen ankondensierten Benzolring enthalten und einbis dreifach gleich oder verschieden substituiert sein und über das Heteroatom oder ein Kohlenstoffatom gebunden sein. Beispielsweise sind die folgenden 5-und 6-Ringheteroaromaten jeweils geeignet: Imidazol, Indol, Isoxazol, Isothiazol, Furan, Oxadiazol, Oxazol, Pyrazin, Pyridazin, Pyrimidin, Pyridin, Pyrazol, Pyrrol, Tetrazol, Thiazol, Triazol, Thiophen, Thiadiazol, Benzimidazol, Benzofuran, Benzoxazol, Isochinolin, Chinolin. Bevorzugt sind 5-oder 6-gliedrige Heteroaromaten mit 1 bis 2 Stickstoff-, Sauerstoff- oder Schwefelatomen und insbesondere Furanyl und Thienyl. Als Substituenten der Heteroarylreste sind insbesondere NO₂, CN, Halogen, C₁₋₆-Alkyl und CF₃ geeignet.

Als Heterocyclus NR¹⁶R¹⁹ seien beispielsweise Piperidin, Pyrrolidin, Morpholin, Thiomorpholin, Hexahydroazepin oder Piperazin genannt. Der Heterocyclus kann 1 - 3-fach substituiert sein mit C₁₋₄-Alkyl oder einem gegebenenfalls mit Halogen substituerten Phenyl-, Benzyl- oder Benzoylrest. Insbesondere geeignet sind 6-gliedrige gesättigte Heterocyclen, die ein weiteres Heteroatom enthalten konnen und die 1- oder 2-fach substituiert sein können. Beispielsweise seien genannt N-Methyl-piperazin, 2,6-Dimethylmorpholin, Phenylpiperazin oder 4-(4-Fluorbenzoyl)-piperidin.

Der Acylrest leitet sich von geradkettigen oder verzweigten aliphatischen C₁-C₆-Carbonsäuren ab wie beispielsweise Ameisensäure, Essigsäure, Propionsäure, Buttersäure, Trimethylessigsäure oder Capronsäure oder von bekannten Benzolsulfonsäuren, die mit Halogen oder C₁₋₄-Alkyl substituiert sein können, sowie von C₁₋₄-Alkansulfonsäuren wie beispielsweise Methansulfonsäure, p-Toluolsulfonsaure Bevorzugt seien Alkanoyle genannt.

Als bevorzugte Bedeutungen von R¹ und R² seien Wasserstoff, C₁₋₆-Alkyl und Hydroxy genannt.

R³ bedeutet vorzugsweise Alkylen mit 1 bis 5 Kohlenstoffatomen, bei dem eine CH₂-Gruppe durch Sauerstoff oder Schwefel ersetzt sein kann. Beispielsweise genannt seien -CH₂-O-CH₂-, -CH₂-S-CH₂-, -( CH₂)₃-, -(CH₂)₄- und -(CH₂)₅-.

Bevorzugte Ausführungsformen für R⁴, R⁵, R⁶ und R⁷ sind:
a) Wasserstoff,
b) Halogen,
c) SR¹⁶,
d) OR¹⁶,
e) COOR¹⁶,
f) COR¹⁶,
g) CONR¹⁶R¹⁷,
h) NR¹⁶R¹⁹,
i) C₁₋₆-Alkyl, das gegebenenfalls substituiert ist mit Halogen, COOR¹⁶ oder Phenyl,
j) C₂₋₆-Alkenyl, gegebenenfalls substituiert mit Phenyl oder Halogen,
k) C₂₋₆-Alkinyl, gegebenenfalls substituiert mit Phenyl oder Halogen,
l) Phenyl, das gegebenenfalls substituiert ist mit Halogen, CN, C₁₋₄-Alkyl, SR¹⁶ oder OR¹⁶,
m) 5-6-gliedriges Heteroaryl mit 1 bis 4 N-, O- oder S-Atomen, das mit Halogen, NO₂, Cyano, C₁₋₄-Alkyl oder CF₃,
n) CN,
o) NO₂,
p) CF₃,
q) OCF3.

Bevorzugte Bedeutungen von R¹², R¹³, R¹⁴, R¹⁷ und R¹⁹ sind Wasserstoff und C₁₋₆-Alkyl. Insbesondere sind 1-2 Substituenten R⁴, R⁵, R⁶ oder R⁷ vorhanden, die nicht Wasserstoff bedeuten.

Die Erfindung betrifft auch die Verwendung der erfindungsgemäßen Verbindungen zur Herstellung eines Arzneimittels zur Behandlung und Prävention von Erkrankungen, die durch die Wirkung von Stickstoffmonoxid in pathologischen Konzentrationen hervorgerufen werden. Dazu zählen neurodegenerative Erkrankungen, inflammatorische Erkrankungen, Autoimmunerkrankungen, Herz-Kreislauf-Erkrankungen.

Beispielsweise seien genannt: Cerebrale Ischaemie, Hypoxie und andere neurodegenerative Erkrankungen, die mit Entzündungen in Verbindung gebracht werden wie Multiple Sklerose, Amyotrophe Lateralsklerose und vergleichbare sklerotische Erkrankungen, Morbus Parkinson, Huntington's Disease, Korsakoffs Disease, Epilepsie, Erbrechen, Schlafstörungen, Schizophrenie, Depression, Migräne, Hypoglykämie, Demenz wie z.B. Alzheimersche Krankheit, HIV-Demenz und präsenile Demenz.

Ferner eignen sie sich zur Behandlung von Krankheiten des Herz-Kreislauf-Systems und zur Behandlung autoimmuner und/oder inflammatorischer Erkrankungen wie Hypotension, ARDS (adult respiratory distress syndrome), Sepsis oder Septischer Schock, Rheumatoider Arthritis, Osteoarthritis, von insulinabhängiger Diabetes Mellitus (IDDM), entzündlicher Erkrankung des Beckens/Darms (bowel disease), von Meningitis, Glomerulonephritis, akute und chronische Lebererkrankungen, Erkrankungen durch Abstoßung (beispielsweise allogene Herz-,Nieren- oder Lebertransplantationen) oder entzündlichen Hautkrankheiten wie Psoriasis und andere. Auf Grund ihres Wirkprofils eignen sich die erfindungsgemäßen Verbindungen sehr gut zur Inhibition der neuronalen NOS.

Zur Verwendung der erfindungsgemäßen Verbindungen als Arzneimittel werden diese in die Form eines pharmazeutischen Präparats gebracht, das neben dem Wirkstoff für die enterale oder parenterale Applikation geeignete Träger-, Hilfsund/oder Zusatzstoffe enthält. Die Applikation kann oral oder sublingual als Feststoff in Form von Kapseln oder Tabletten oder als Flüssigkeit in Form von Lösungen, Suspensionen, Elixieren, Aerosolen oder Emulsionen oder rektal in Form von Suppositorien oder in Form von gegebenenfalls auch subcutan intramuskulär oder intravenös anwendbaren Injektionslösungen oder topisch oder intrathekal erfolgen. Als Hilfsstoffe für die gewünschte Arzneimittelformulierung sind die dem Fachmann bekannten inerten organischen und anorganischen Tragermaterialien geeignet wie z.B. Wasser, Gelantine, Gummmi arabicum, Milchzucker, Stärke. Magnesiumstearat, Talk, pflanzliche Öle, Polyalkylenglykole usw. Gegebenenfalls können darüber hinaus Konservierungs-, Stabilisierungs-, Netzmittel, Emulgatoren oder Salze zur Veränderung des osmotischen Druckes oder Puffer enthalten sein.

Für die parenterale Anwendung sind insbesondere Injektionslösungen oder Suspensionen, insbesondere wäßrige Lösungen der aktiven Verbindungen in polyhydroxyethoxyliertem Rizinusöl, geeignet.

Als Trägersysteme können auch grenzflächenaktive Hilfsstoffe wie Salze der Gallensäuren oder tierische oder pflanzliche Phospholipide, aber auch Mischungen davon sowie Liposome oder deren Bestandteile verwendet werden.

Für die orale Anwendung sind insbesondere Tabletten, Dragees oder Kapseln mit Talkum und/oder Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt ist.

Die Dosierung der Wirkstoffe kann je nach Verabfolgungsweg, Alter und Gewicht des Patienten, Art und Schwere der zu behandelnden Erkrankung und ähnlichen Faktoren variieren. Die tägliche Dosis beträgt 1-2000 mg, vorzugsweise 20-500 mg, wobei die Dosis als einmal zu verabreichende Einzeldosis oder unterteilt in 2 oder mehreren Tagesdosen gegeben werden kann.

Die NOS-inhibitorische Wirksamkeit der Verbindungen der Formel (I) und deren physiologisch verträglicher Salze kann nach den Methoden von Bredt und Snyder in Proc. Natl. Acad. Sci. USA (1989) 86, 9030-9033 bestimmt werden. Für die bNOS-Inhibition der Beispiele 21 (4-Amino-7-(4-morpholinyl)-2,3,3a,9b-tetrahydro-1H-cyclopenta[c]chinolin) und 22 (4-Amino-7-(methoxycarbonylethyl)-2,3,3a,9b-tetrahydro-1H-cyclopenta[c]chinolin) ergeben sich IC₅₀ = 1.4 µM und 1.1 µM.

Die Herstellung der erfindungsgemäßen Verbindungen erfolgt dadurch, daß man eine Verbindung der Formel (II) oder deren Salz 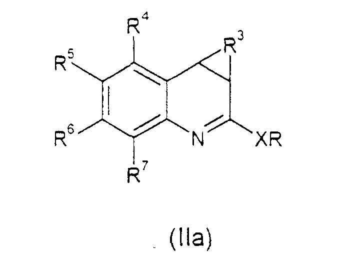 oder 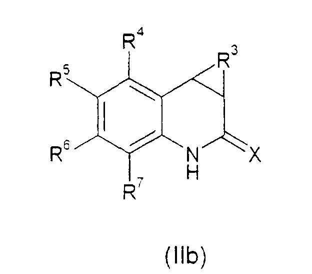 worin R³ bis R⁷ die obige Bedeutung haben, R Methyl oder Ethyl und X Sauerstoff oder Schwefel ist, mit Ammoniak, primären oder sekundären Aminen, Hydroxylamin und seinen Derivaten oder Hydrazin und seinen Derivaten umsetzt und gewünschtenfalls anschließend die Isomeren trennt oder die Salze bildet.

Die Umsetzung mit Ammoniak gelingt unter Druck in Autoklaven bei Ammoniaküberschuß bei tiefen Temperaturen (- 78 °C) oder durch Rühren in mit Ammoniak gesättigten Methanol. Bevorzugt werden Thiolactame umgesetzt. Wird mit Aminen umgesetzt, so stellt man aus dem Lactam oder Thiolactam zunächst den Iminoether oder Iminothioether als Zwischenverbindung dar (z.B. mit Methyliodid oder Dimethylsulfat) und setzt diesen mit oder ohne Isolierung mit den entsprechenden Aminen oder deren Salzen um.

Die Isomerengemische können nach üblichen Methoden wie beispielsweise Kristallisation, Chromatographie oder Salzbildung in die Enantiomeren bzw. E/Z-Isomeren aufgetrennt werden. Die Enantiomeren können auch durch Chromatographie an chiralen Phasen sowie durch stereoselektive Synthese erhalten werden.

Die Herstellung der Salze erfolgt in üblicher Weise, indem man eine Lösung der Verbindung der Formel (I) mit der äquivalenten Menge oder einem Überschuß einer Säure, die gegebenenfalls in Lösung ist, versetzt und den Niederschlag abtrennt oder in üblicher Weise die Lösung aufarbeitet.

Soweit die Herstellung der Ausgangsverbindungen nicht beschrieben wird, sind diese bekannt und käuflich oder analog zu bekannten Verbindungen oder nach hier beschriebenen Verfahren herstellbar.

Thiolactame der Formel (IIb. X = S) erhält man beispielsweise aus Lactamen mit Phosphorpentasulfid (P₄S₁₀) oder 2,4-Bis(4-methoxyphenyl)-1,3,2,4-dithiaphosphetan-2,4-disulfid (Lawessons Reagenz) in geeigneten Lösungsmitteln. Verbindungen der Formell (IIa) können beispielsweise durch Umsetzung mit Meerwein-Reagenz (Trimethyloxoniumtetrafluoroborat) erhalten werden.

Die Herstellung der Verbindungen der Formel (IIb, X = O) erfolgt nach der dem Fachmann bekannten Weise Sie kann beispielsweise dadurch erfolgen, daß man eine Verbindung der Formel (III). 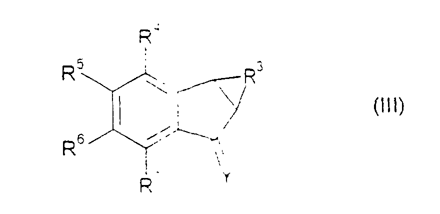 worin R³ bis R⁷ die obige Bedeutung haben und Y = O ist, nach Überführung in das Oxim (Y = NOH), beispielsweise mit einem Hydroxylammoniumsalz und Natriumacetat, einer Beckmann-Umlagerung (R. E. Gawley, *Org. Reactions* **1988**, 35, 1), beispielsweise in Polyphosphorsäure (vgl. K. Hino, Y. Nagai, H. Uno, *Chem. Pharm. Bull.* **1988**, *36*. 2386) unterwirft.
Ein anderer Syntheseweg geht von einer Verbindung der Formel (IV) aus, 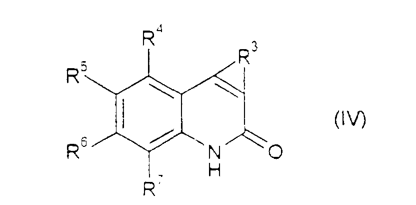 die mit einem Alkali- oder Erdalkalimetal oder einem Amalgam derselben in Alkohol zu Lactam (II) reduziert wird (vgl. B.K. Blount, W.H. Perkin, S.G.P. Plant, *J. Chem. Soc.* **1929,** 1975; R. Brettle, S.M. Shibib, *J.Chem. Soc. Perkin Trans 1,* **1981,** 2912).

Die Herstellung der Indanone vom Typ (III) erfolgen in der dem Fachmann bekannten Weise, z. B. nach W. Baker, P.G. Jones, *J. Chem. Soc.* **1951**, 787; S. Ohta, M. Yamashita, K. Arita, T: Kajiura, I. Kawasaki, K. Noda, M. Izumi, *Chem. Pharm. Bull.* **1995,** *43*, 1294; C. Santelli-Rouvier, M. Santelli, *Synthesis* **1983**,429.

Die Herstellung der Chinolone vom Typ (IV) erfolgt in der dem Fachmann bekannten Weise, z. B. nach B.K. Blount, W.H. Perkin, S.G.P. Plant, *J. Chem. Soc.* **1929**, 1975; W. Ried, W. Käppeler, *Liebigs Ann. Chem.* **1965**, *688*, 177; L.A. White, R.C. Storr, *Tetrahedron* **1996**, *52*, 3117.

Die Herstellung der Verbindung der Formel (III) kann beispielsweise dadurch erfolgen, daß man einen Aromaten (V) mit einem aktivierten Säurederivat, wie beispielsweise einem Säurechlorid (Z = Cl) oder Anhydrid (Z = OCOR) in Gegenwart einer Lewissäure, wie beispielsweise AlCl₃, SnCl₄, ZnCl₂, SbCl₅, FeCl₃, BF₃-Etherat, in einem inerten Lösungsmittel, wie beispielsweise Dichlormethan, Dichlorethan oder Benzol, bei 0°C bis zur Siedetemperatur des entsprechenden Lösungsmittels umsetzt (s. z.B. W. Baker, P.G. Jones, *J. Chem. Soc.* **1951**, 787). Alternativ können die nach dem Fachmann bekannten Wegen hergestellten Ketone der Formel (VII) (z.B. nach S. Ohta, M. Yamashita, K. Arita, T: Kajiura, I. Kawasaki, K. Noda, M. Izumi, *Chem. Pharm. Bull.* **1995**, *43*, 1294) mit Brönstedtsäuren, wie beispielsweise Schwefelsäure, p-Toluolsulfonsäure, Methansulfonsäure, Phosphorsäure oder Polyphosphorsäure (vgl. C. Santelli-Rouvier, M. Santelli, *Synthesis* **1983**, 429), cyclisiert werden.

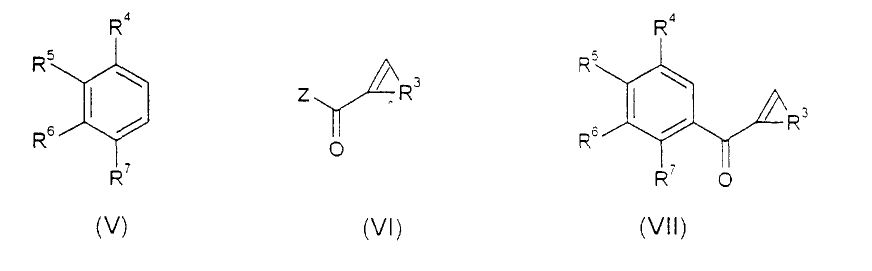

Die Herstellung der Verbindung der Formel (IV) kann beispielsweise dadurch erfolgen, daß ein beta-Ketoamid der Formel (VIII) oder ein Derivat desselben mit einer Säure, beispielsweise Schwefelsäure, Phosphorsäure, Polyphosphorsäure oder Trifluoressigsäure oder Methansulfonsäure, behandelt wird (z.B. B.K. Blount, W.H. Perkin, S.G.P. Plant, *J*. *Chem. Soc.* **1929**, 1975; W. Ried, W. Käppeler, Liebigs *Ann. Chem.* **1965,** *688*, 177).

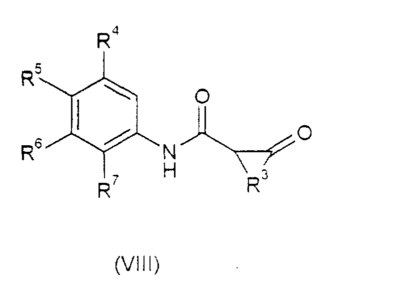

Die Einführung der Substituenten R⁴-R⁷ kann auf der Stufe der Verbindungen (III) und (IV) erfolgen.

An den Vorstufen werden gewünschtenfalls Sulfide oxidiert, Ester verseift, Säuren verestert, Hydroxygruppen verethert oder acyliert, Amine acyliert, alkyliert, diazotiert, halogeniert, NO₂ eingeführt oder reduziert, mit Isocyanaten oder Isothiocyanaten umgesetzt, die Isomeren getrennt oder die Salze gebildet.

Die Verseifung einer Estergruppe kann basisch oder sauer erfolgen, indem man bei Raumtemperatur oder erhöhter Temperatur bis zur Siedetemperatur des Reaktionsgemisches in Gegenwart von Alkalihydroxiden in Ethanol oder anderen Alkoholen oder mittels Säuren wie z.B. Salzsäure hydrolysiert und ggf. Salze der 3,4-Cycloalkanodihydrochinoline weiterverarbeitet.

Die Veresterung der Carbonsäure geschieht in an sich bekannter Weise mit Diazomethan oder dem entsprechenden Alkohol in Säure oder in Gegenwart eines aktivierten Säurederivats. Als aktivierte Säurederivate kommen zum Beispiel Säurechlorid, -imidazolid oder -anhydrid in Frage.

Zusätzlich kann durch elektrophile aromatische Substitution eine Nitrogruppe oder Halogen, insbesondere Brom, eingeführt werden. Dabei entstehende Gemische können in üblicher Weise, auch mittels HPLC, getrennt werden.

Wenn ein Nitril vorliegt, kann dieses nach bekannten Verfahren verseift werden oder in das entsprechende Amin, Tetrazol oder Amidoxim übergeführt werden.

Die Friedel-Crafts-Acylierung wird bei Lactamen vom Typ (IIb, X = O) erfolgreich angewandt; anschließend kann selektiv das Lactam in das Thiolactam übergeführt werden.

Die Einführung einer NO₂-Gruppe gelingt durch eine Reihe von bekannten Nitrierungsmethoden. Beispielsweise kann mit Nitraten oder mit Nitroniumtetrafluoroborat in inerten Lösungsmitteln wie halogenierten Kohlenwasserstoffen oder in Sulfolan oder Eisessig nitriert werden. Möglich ist auch die Einführung z.B. durch Nitriersäure in Wasser, Essigsäure oder konz. Schwefelsäure als Lösungsmittel bei Temperaturen zwischen - 10 °C und 30 °C.

Die Reduktion der Nitrogruppe oder ggf. der Cyanogruppe zur Aminogruppe erfolgt katalytisch in polaren Lösungsmitteln bei Raumtemperatur oder erhöhter Temperatur unter Wasserstoffdruck. Als Katalysatoren sind Metalle wie Raney-Nickel oder Edelmetallkatalysatoren wie Palladium oder Platin gegebenenfalls in Gegenwart von Bariumsulfat oder auf Trägern geeignet. Statt Wasserstoff kann auch Ammoniumformiat oder Ameisensäure in bekannter Weise benutzt werden. Reduktionsmittel wie Zinn(II)chlorid oder Titan(III)chlorid können ebenso verwendet werden wie komplexe Metallhydride, eventuell in Gegenwart von Schwermetallsalzen. Für Nitrogruppen bewährt hat sich die Reduktion mit Zink in Wasser-Ethanol-THF/ Ammoniumchlorid oder Eisen in Essigsäure.

Wird eine einfache oder mehrfache Alkylierung einer Aminogruppe oder einer CH-aciden Kohlenstoffposition gewünscht, so kann nach üblichen Methoden beispielsweise mit Alkylhalogeniden alkyliert werden. Gegebenenfalls ist Schutz der Lactamgruppe als Anion durch ein 2. Equivalent Base oder durch eine passende Schutzgruppe erforderlich.

Die Acylierung der Aminogruppe erfolgt in üblicher Veise beispielsweise mit einem Säurehalogenid oder Säureanhydrid gegebenenfalls in Gegenwart einer Base.

Die Einführung der Halogene Chlor, Brom oder Jod über die Aminogruppe kann beispielsweise auch nach Sandmeyer erfolgen, indem man die mit Nitriten intermediär gebildete Diazoniumsalze mit Cu(I)chlorid oder Cu(I)bromid in Gegenwart der entsprechenden Säure wie Salzsäure oder Bromwasserstoffsäure umsetzt oder mit Kaliumjodid umsetzt.

Neue Verbindungen wurden durch eine oder mehrere der folgenden Methoden charakterisiert: Schmelzpunkt, Massenspektroskopie, Infrarotspektroskopie, Nuklear-magnetische Resonanzspektroskopie (NMR). NMR Spektren wurden mit einem Bruker 400 MHz Gerät gemessen, die (deuterierten) Lösemittel werden jeweils angegeben und wie folgt abgekürzt: CDCl₃ (Chloroform), CD₃OD ([D₄]-Methanol), DMSO ([D_{6]}-Dimethylsulfoxid). Verschiebungen sind in delta und ppm angegeben. Es bedeuten: m (Multiplett, mehrere Signale), s (Singulett), d (Dublett), dd (Doppeldublett usw.), t (Triplett), q (Quartett), H (Wasserstoffprotonen). Ferner bedeuten: THF (Tetrahydrofuran), DMF (N,N-Dimethylformamid), MeOH (Methanol), mL (Milliliter). Alle Lösemittel sind p.A.Qualität, wenn nicht anders vermerkt. Alle Reaktionen werden unter Schutzgas ausgeführt, es sei denn es handelt sich um wäßrige Lösungen. Schmelzpunkte werden in Grad Celsius angegeben und sind nicht korrigiert.

Nachfolgend wird die Darstellung von Vorstufen, Zwischenprodukten und Produkten exemplarisch beschrieben.

### Ausgangsverbindungen

### A) 1,2,3,3a,5,9b-Hexahydrocyclopenta[c]chinolin-4-on

### nach Methode A:

2,3,3a,8a-Tetrahydro-1H-cyclopent[a]inden-8-on (1.06 g, 6.0 mmol) (W. Baker, P.G. Jones, *J. Chem. Soc.* **1951,** 787) werden mit Hydroxylammoniumsulfat (1.96 g, 12.0 mmol) und Natriumacetat (24.0 mmol, 3.28g) in THF-Ethanol-Wasser 1:1:1 (120 mL) gelöst und 5 Tage bei Raumtemperatur gerührt. Das Reaktionsgemisch wird eingeengt und mit Essigester (150 mL) verdünnt, mit ges. NaCl (50 mL) gewaschen, getrocknet (Na₂SO₄) und i. Vak. eingeengt. Der Rückstand wird säulenchromatographisch (SiO₂) mit dem Eluens Hexan-Essigester gereinigt. Ausbeute 0.88 g (78%), Schmp. 118-20°C; das derart erhaltene Oxim (0.65 g, 3.5 mmol) wird in 120°C heißer Polyphosphorsäure (10 mL) gegeben. Der Ansatz wird 30 min bei 120°C gerührt. Nach dem Abkühlen wird er in Wasser aufgenommen (150 mL) und die wäßrige Lösung mit Essigester extrahiert (3x 150 mL) Die vereinigten Essigester-Extrakte werden getrocknet (Na₂SO₄) und i. Vak eingeengt. Der Rückstand wird säulenchromatographisch (SiO₂) mit dem Eluens Hexan-Essigester gereinigt. Ausbeute: 202 mg (31 %), Schmp 133 - 5°C

### nach Methode B:

1,2,3,5-Tetrahydrocyclopenta[c]chinolin-4-on (410 mg, 2.21 mmol) (W. Ried, W. Käppeler, *Liebigs Ann. Chem* **1965** *688*, 177) wird in Methanol (25 mL) gelöst und mit Magnesium (538 mg. 22.1 mmol) versetzt. Nach 3 h Rühren bei Raumtemperatur wird der Ansatz filtriert der Filterrückstand mit Essigester gewaschen, und die vereinigten Filtrate eingeengt. Säulenchromatographische (SiO₂) Reinigung mit dem Eluens Hexan-Essigester liefern 290 mg (71 %) des Produkts.
¹H-NMR (CDCl₃): 1.60-1.80 (m, 3H, 2 03-2.20 (m, 2H), 2.26-2.40 (m, 1 H), 2.96 (td, 1H), 3.20-3.32 (m, 1H), 6.78 (dd, 1H), 7.00 (td, 1H), 7.17 (td, 1H), 7.21 (dd, 1H), 8.32 (br. s, 1H).
MS (EI) m/e = 187 (M⁺)

### gemäß Methode A werden synthetisiert

### 6a,7,8,9,10,10a-Hexahydro-5H-phenanthridin-6-on

Aus 1,2,3,4,4a,9a-Hexahydrofluoren-9-on (2.7 g, 14.5 mmol) wird 1,2,3,4,4a,9a-Hexahydrofluoren-9-oxim (3.18 g) gewonnen. Davon werden 2.0 g mit Polyphosphorsäure (20 mL) in 0.22 g (11%) Produkt umgewandelt.

Schmp. 212-3 °C (Säulenchromatographie an Kieselgel, Eluens: Essigester-Hexan)
¹H-NMR (CDCl₃): 1.40 - 1.85 (m, 7H), 2.38 (m, 1H), 2.85 (m, 1H), 2.98 (m, 1H), 6.77 (dd, 1H), 7.03 (td, 1H), 7.19 (dd, 1H), 7.21 (td, 1H), 8.04 (br. s, 1H).
MS (EI) m/e = 201 (M⁺)
Als polareres Nebenprodukt fällt 1,2,3,4,4a,10b-Hexahydro-5H-phenanthridin-6-on an.

### 1,2,3,4,5,5a,7,11b-Octahydrocyclohepta[c]chinolin-6-on

Aus 5,6,7,8,9,9a-Hexahydro-4bH-benz[a]azulen-10-on (2.89 g) wird 5,6,7,8,9,9a-Hexahydro-4bH-benz[a]azulen-10-oxim (2.9 g) gewonnen. Mit Polyphosphorsäure (20 mL) ergeben sich daraus 0.33 g Produkt.
¹H-NMR (CDCl₃): 1.40 - 1.90 (m, 9H), 2.12 (m, 1H), 2.88 (m, 1H), 3.09 (m, 1H), 6.75 (dd, 1H), 7.01 (td, 1H), 7.16 (td, 1H), 7.18 (dd, 1H), 8.05 (br. s, 1H).
MS (EI) m/e = 215 (M⁺)
Als polareres Nebenprodukt fällt 1,2,3,4,5,5a,7,11b-Octahydrocyclohepta[c]isochinolin-7-on an.

### B) 1,2,3,3a,5,9b-Hexahydrocyclopenta[c]chinolin-4-thion

1,2,3,3a,5,9b-Hexahydrocyclopenta[c]chinolin-4-on (153 mg, 0.82 mmol) und Lawessons Reagenz (363 mg, 0.9 mmol) werden in Dimethoxyethan (20 mL) bei Raumtemperatur gerührt. Nach 3 h wird eine weitere Portion von Lawessons Reagenz (363 mg, 0.9 mmol) dazugegeben. Nach 3 h wird der Ansatz eingeengt und der Rückstand säulenchromatographisch an Kieselgel gereinigt (Eluens: Hexan-Essigester).
Ausbeute: 140 mg (84%), Schmp. 138 - 40°C
¹H-NMR (CDCl₃): 1.60 - 1.80 (m, 2H), 1.93 (m, 1H), 2.10 - 2.40 (m, 3H), 3.32 (m, 2H), 6.86 (dd, 1H), 7.12 (td, 1H), 7.20 (td, 1H), 7.25 (dd, 1H), 9.87 (br. s, 1H).
MS (EI) m/e = 203 (M⁺)

### analog werden synthetisiert:

### 6a,7,8,9,10,10a-Hexahydro-5H-phenanthridin-6-thion

Aus 6a,7,8,9,10,10a-Hexahydro-5H-phenanthridin-6-on (44 mg, 0.22 mmol) wird 26 mg (54%) Produkt erhalten
¹H-NMR (CDCl₃): 1.45 - 1.82 (m, 6H), 1.99 (m, 2H), 3.05 - 3.20 (m, 2H), 6.85 (dd, 1H), 7.13 (td, 1H), 7.16 (td, 1H), 7.23 (dd, 1H), 9.73 (br. s, 1H).

### 1,2,3,4,5,5a,7,11b-Octahydrocyclohepta[c]chinolin-6-thion

Aus 1,2,3,4,5,5a,7,11b-Octahydrocyclohepta[c]chinolin-6-on (100 mg, 0.46 mmol) wird 100 mg (94%) Produkt erhalten.
Schmp. 112°C
¹H-NMR (CDCl₃): 1.40 - 2.10 (m, 10H), 3.13 - 3.29 (m, 2H), 6.85 (dd, 1H), 7.10 - 7.25 (m, 3H), 9.76 (br. s, 1H).
MS (EI) m/e=231 (M⁺)

### Beispiel 1

### 4-Amino-2,3,3a,9b-tetrahydro-1H-cyclopenta[c]chinolin

1,2,3,3a,5,9b-Hexahydrocyclopenta[c]chinolin-4-thion (110 mg, 0.54 mmol) wird in 7 M methanolischer Ammoniaklösung (50 ml) 15 h bei Raumtemperatur gerührt. Der Ansatz wird i. Vak. eingeengt. Der Rückstand wird säulenchromatographisch an Kieselgel mit Dichlormethan-Methanol-konz. NH₄OH als Eluens gereinigt.
Ausbeute: 100 mg (99%)
¹H-NMR (CDCl₃): 1.41 - 1.98 (m, 4H), 2.11 - 2.25 (m, 2H), 2.83 (q, 1H), 3.30 (q, 1H), 4.44 (br., 2H), 6.96 - 7.05 (m, 2H), 7.12 - 7,29 (m, 2H).
MS (EI) m/e = 186 (M⁺)

### Beispiel 2

### 6-Amino-6a,7,8,9,10,10a-hexahydrophenanthridin

Analog zu Beispiel 1 wird 6a,7,8,9,10,10a-Hexahydro-5H-phenanthridin-6-thion (26 mg, 0.12 mmol) in 10 mg (42%) Produkt übergeführt.
¹H-NMR (CDCl₃): 1.20 - 1.80 (m, 6H), 1.90 - 2.23 (m, 2H), 2.43 - 3.05 (m, 2H), 5.35 (br., 2H), 7.00 (m, 2H), 7.18 (m, 2H).
MS (EI) m/e = 200 (M⁺)

### Beispiel 3

### 6-Amino-2,3,4,5,5a,11b-hexahydro-1H-cyclohepta[c]chinolin

Analog zu Beispiel 1 wird 1,2,3,4,5,5a,7,11b-Octahydrocyclohepta[c]chinolin-6-thion (90 mg, 0.39 mmol) in 70 mg (84%) Produkt übergeführt.
¹H-NMR (CD₃OD): 1.30 - 1.78 (m, 9H), 2.00 (m, 2H), 3.11 (t, 1H), 6.92 (dd, 1H), 6.96 (td, 1H), 7.07 (td, 1H), 7.17 (dd, 1H).
MS (EI) m/e = 214 (M⁺)

### Beispiel 4

### 4-Amino-7-nitro-2,3,3a,9b-tetrahydro-1H-cyctopenta[c]chinolin

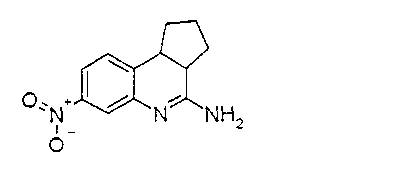

### 6-Nitro-2,3,3a,8a-tetrahydro-1H-cyclopent[a]inden-8-on und 5-Methyl-6-nitro-2,3,3a,8a-tetrahydro-1H-cyclopent[a]inden-8-on

Cyclopenten-1-carbonsäure (3.92 g, 35 mmol) wird in 35 mL Thionylchlorid 30 min unter Rückfluß erhitzt. Die Lösung wird i. Vak. eingeengt und Thionylchloridreste mit Toluol azeotrop entfernt. Der Rückstand wird in Benzol aufgenommen, mit Aluminiumtrichlorid (23.33 g, 175 mmol) versetzt, 1 h unter Rückfluß erhitzt und 1 Woche bei Raumtemp. belassen. Der Ansatz wird auf Eis gegossen und mit Ether extrahiert. Die organische Phase wird mit Wasser gewaschen, getrocknet (Na₂SO₄) und i. Vak. eingeengt. Säulenchromatographie an Kieselgel mit Ether-Hexan lieferte 3.85 g eines 2:1-Gemisches von 2,3,3a,8a-Tetrahydro-1H-cyclopent[a]inden-8-on^{*} und 5-Methyl-2,3,3a,8a-tetrahydro-1H-cyclopent[a]inden-8-on^{#}: ¹H-NMR (CDCl₃): δ = 1.06 - 1.25 (m, 1H), 1.61 (m, 1H), 1.79 - 2.12 (m, 4H), 2.45^{#} (s, ∼ ³/₃ H), 3.03 - 3.12 (m, 1H), 3.72^{#} (t) und 3.78* (t, ∑ 1H); 7.16^{#} (d), 7.28^{#} (s), 7.37* (t), 7.49* (d), 7.62^{#} (d), 7.62* (t) und 7.68* (d, ∑ ∼ 4H). Dieses Gemisch wird bei 5 °C langsam mit 20 mL Nitriersäure (zubereitet aus 10 mL 96 proz. Schwefelsäure und 10 mL 65 proz. Salpetersäure) versetzt. Nach 45 min bei 5 - 10 °C wird der Ansatz auf Eis gegossen und mit Ether extrahiert. Die vereinigten Etherextrakte werden mit ges. NaHCO₃ gewaschen, getrocknet (Na₂SO₄) und i. Vak, eingeengt.

Säulenchromatographie an Kieselgel mit Ether-Hexan liefert 3.76 g eines 2:1-Gemisches von 6-Nitro-2,3,3a,8a-tetrahydro-1H-cyclopent[a]inden-8-on* und 5-Methyl-6-nitro-2,3,3a,8a-tetrahydro-1H-cyclopent[a]inden-8-on^{#}.
¹H-NMR (CDCl₃): δ = 1.09 - 1.26 (m, 1H), 1.70 (m, 1H), 1.82 - 2.19 (m, 4H), 2.68^{#} (s, ∼ ³/₃ H), 3.16^{#} (ddd) und 3.22* (ddd, Σ 1H), 3.81^{#} (t) und 3.90* (t, ∑ 1H); 7.46^{#} (s, ¹/₃ H), 7.67* (dd, ²/₃ H), 8.23^{#} (s, ¹/₃ H), 8.47* (dd, ²/₃ H), 8.51* (d, ²/₃ H).

### 7-Nitro-1,2,3,3a,5,9b-hexahydrocyclopenta[c]chinolin-4-on 8-Methyl-7-nitro-1,2,3,3a,5,9b-hexahydrocyclopenta[c]chinolin-4-on

Das Gemisch aus 6-Nitro-2,3,3a,8a-tetrahydro-1H-cyclopent[a]inden-8-on und 5-Methyl-6-nitro-2,3,3a,8a-tetrahydro-1H-cyclopent[a]inden-8-on (3.76 g) wird mit Hydroxylammoniumsulfat (5.58 g, 34 mmol) und Natriumacetat (9.25 g, 68 mmol) in THF-Ethanol-Wasser 1:1:1 (180 mL) gelöst. Der Ansatz wird 6 h bei 60 °C gerührt, 3 Tage bei Raumtemp. belassen, eingeengt, mit Ether verdünnt, mit ges. NaCl gewaschen, getrocknet (Na₂SO₄) und i. Vak. eingeengt. Der Rückstand wird an Kieselgel mit dem Eluens Ether-Hexan gereinigt. Ausbeute: 3.76 g; das Oximgemisch wird bei 120°C in Polyphosphorsäure gelöst. Der Ansatz wird 4 h bei 120°C gerührt, nach Abkühlung auf Eiswasser gegossen und mit Essigester extrahiert. Die vereinigten Extrakte werden getrocknet (Na₂SO₄) und i. Vak. eingeengt. Der Rückstand wird an Kieselgel mit Essigester-Hexan vorgereinigt und das Gemisch anschließend mittels präparativer HPLC (LC 50 Novapak-Säule, Eluens: Methanol-wäßrige Ammoniumacetatlösung) aufgetrennt:
91 mg 7-Nitro-1,2,3,3a,5,9b-hexahydrocyclopenta[c]chinolin-4-on :
¹H-NMR (CDCl₃): δ = 1.57 - 1.86 (m, 3H), 2.10 - 2.25 (m, 2H), 2.41 (m, 1H), 3.03 (td, 1H), 3.36 (q, 1H), 7.38 (d, 1H), 7.66 (d, 1H), 7.88 (dd, 1H), 8.58 (br. s, 1H);
60 mg 8-Methyl-7-nitro-1,2,3,3a,5,9b-hexahydrocyclopenta[c]chinolin-4-on:
¹H-NMR (CDCl₃): δ = 1.49 - 1.81 (m, 3H), 2.09 - 2.22 (m, 2H), 2.38 (m, 1H), 2.56 (s, 3H), 2.98 (td, 1H), 3.29 (q, 1H), 7.16 (s, 1H), 7.45 (s, 1H), 8.40 (br. s, 1H).

### 7-Nitro-1,2,3,3a,5,9b-hexahydrocyclopenta[c]chinolin-4-thion

7-Nitro-1,2,3,3a,5,9b-hexahydrocyclopenta[c]chinolin-4-on (91 mg, 0.30 mmol) wird mit Lawessons Reagenz (170 mg, 0.43 mmol) 6 h bei Raumtemp. gerührt. Der Ansatz wird i. Vak. eingeengt und der Rückstand an Kieselgel mit Toluol gereinigt: 52 mg (54%) Produkt.
¹H-NMR ([D₆]-DMSO):δ = 1.47 - 1.77 (m, 3H), 2.09 - 2.24 (m, 3H), 3.40 (m, 2H), 7.57 (d, 1H), 7.91 (dd, 1H), 8.00 (d, 1H), 12.40 (br. s, 1H).

### 4-Amino-7-nitro-2,3,3a,9b-tetrahydro-1H-cyclopenta[c]chinolin

7-Nitro-1,2,3,3a,5,9b-hexahydrocyclopenta[c]chinolin-4-thion (47 mg, 0.19 mmol) werden in 7 N methanolischem Ammoniak (10 mL) 15 h bei Raumtemp. gerührt. Der Ansatz wird i. Vak. eingeengt. Der Rücksatnd wird an Kieselgel mit Dichlormethan-2-Propanol/Methanol gereinigt: 21 mg (48%) Produkt.
¹H-NMR ([D_{6]}-DMSO)- δ = 1.45 - 1 66 (m, 3H), 1.87 (m, 1H), 2.06 - 2.22 (m, 2H), 2.77 (m, 1H), 3.33 (m, 1H), 7 06 (br., 2H), 7.37 (d, 1H), 7.52 (s, 1H), 7.67 (d, 1H);
MS (EI) m/e = 231 (M⁺).

### Beispiel 5

### 4-Amino-8-methyl-7-nitro-2,3,3a, 9b-tetrahydro-1H-cyclopenta[c]chinolin

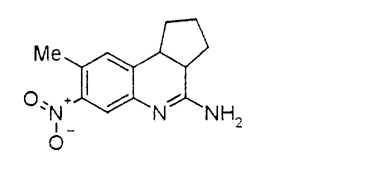

### 8-Methyl-7-nitro-1,2,3,3a,5,9b-hexahydrocyclopenta[c]chinolin-4-thion

Wie für Beispiel 4 beschrieben wird 8-Methyl-7-nitro-1,2,3,3a,5,9b-hexahydrocyclopenta[c]chinolin-4-on (60 mg, 0.24 mmol) mit Lawessons Reagenz (105 mg, 0.26 mmol) zu 47 mg (75%) Produkt umgesetzt.
¹H-NMR (CDCl₃): δ = 1.66 - 1 79 (m 2H), 1.88 (m, 1H), 2.16 - 2.41 (m, 3H), 2.59 (s, 3H), 3.32 (m, 2H), 7.22 (s, 1H) 7.52 (s, 1H), 9.61 (br. s, 1H).

### 4-Amino-7-nitro-2,3,3a,9b-tetrahydro-1H-cyclopenta[c]chinolin

Wie für Beispiel 4 beschrieben, wird 8-Methyl-7-nitro-1,2,3,3a,5,9b-hexahydrocyclopenta[c]chinolin-4-thion (42 mg, 0.16 mmol) mit 7N methanolischer Ammoniaklösung (10 mL) zu 30 mg (77%) Produkt umgesetzt.
¹H-NMR (CDCl₃): δ = 1.44 - 1.61 (m. 2H), 1.83 - 1.94 (m, 2H), 2.03 - 2.21 (m, 2H), 2.50 (s, 3H), 2.91 (q, 1H), 3.23 (q, 1H), 4.93 (br., 2H), 7.05 (s, 1H), 8.06 (s, 1H);
MS (EI) m/e = 245 (M⁺).

### Beispiel 6

### 4-Amino-8-nitro-2,3,3a,9b-tetrahydro-1H-cyclopenta[c]chinolin

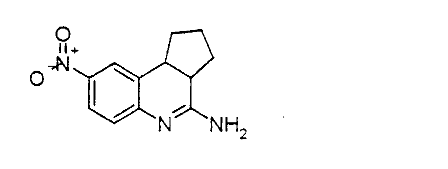

### 8-Nitro-1,2,3,3a,5,9b-hexahydrocyclopenta[c]chinolin-4-on

65 proz. Salpetersäure (0.33 mL) wird bei 0 °C mit 96 proz. Schwefelsäure (0.44 mL) versetzt. Dazu wird eine Lösung von 1,2,3,3a,5,9b-Hexahydrocyclopenta[c]chinolin-4-on (1.9 g, 5.3 mmol) in Dichlormethan (5 mL) getropft. Nach 1 h bei Raumtemp. wird der Ansatz auf Eiswasser gegossen, der Feststoff abfiltriert und mit Wasser gewaschen. Säulenchromatographische Reinigung (SiO₂) des Filterrückstands mit Essigester-Hexan liefert 0.65 g (53%) Produkt.
¹H-NMR (CDCl₃): δ = 1.65 - 1.82 (m, 3H), 2.13 - 2.25 (m, 2H), 2.36 (m, 1H), 3.04 (td, 1H), 3.38 (q, 1H), 6.91 (d, 1H), 8.09 (dd, 1H), 8.15 (d, 1H), 9.07 (br. s, 1H).

### 8-Nitro-1,2,3,3a,5,9b-hexahydrocyclopenta[c]chinolin-4-thion

Wie für Beispiel 4 beschrieben, wird 8-Nitro-1,2,3,3a,5,9b-hexahydrocyclopenta[c]chinolin-4-on (100 mg, 0.43 mmol) mit Lawessons Reagenz (470 mg, 1.16 mmol) zu 100 mg (94%) Produkt umgesetzt.
¹H-NMR ([D₆]-DMSO):δ = 1.51 - 1.78 (m, 3H), 2.09 - 2.28 (m, 3H), 3.47 (m, 2H), 7.31 (d, 1H), 8.14 (dd, 1H), 8.20 (d, 1H), 12.62 (br. s, 1H).

### 4-Amino-8-nitro-2,3,3a,9b-tetrahydro-1H-cyclopenta[c]chinolin

Wie für Beispiel 4 beschrieben, wird 8-Nitro-1,2,3,3a,5,9b-hexahydrocyclopenta[c]chinolin-4-thion (100 mg, 0.40 mmol) mit 7N methanolischer Ammoniaklösung (15 mL) zu 70 mg (76%) Produkt umgesetzt. ¹H-NMR ([D₆]-DMSO): δ = 1.49 - 1.68 (m, 3H), 1.87 (m, 1H), 2.01 - 2.28 (m, 2H), 2.75 (q, 1H), 3.32 (q, 1H), 6.90 (d, 1H), 7.46 (br., 2H), 7.92 (dd, 1H), 7.99 (d, 1H).

### Beispiel 7

### 4-Amino-2-oxa-2,3,3a,9b-hexahydro-1H-cyclopenta[c]chinolin

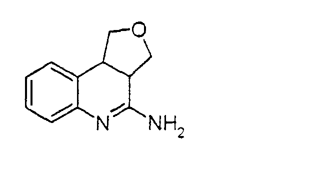

### 3-(Trifluormethylsulfonyloxy)-2,5-dihydrofuran-4-carbonsäure-methylester

Eine Lösung von Tetrahydrofuran-3-on-4-carbonsäure-methylester (P. Dowd, S.-C. Choi, *Tetrahedron* **1991**, *47*, 4847) (2.88 g, 20 mmol) in Dichlormethan (200 mL) wird bei -78 °C mit Diisopropyl(ethyl)amin (3.96 mL, 23.2 mmol) versetzt. Nach 10 min wird Trifluormethansulfonsäureanhydrid (3.88 mL, 23.2 mmol) langsam dazugetropft. Der Ansatz wird auf Raumtemp. erwärmt, 2 h gerührt und eingeengt. Der Rückstand wird säulenchromatographisch (SiO₂) mit Essigester-Hexan gereinigt: 3.62 g (66%) Produkt.
¹H-NMR (CDCl₃): δ = 3.85 (s, 3H), 4.80 (t, .2H), 4.92 (t, 2H).

### 2-Oxa-1,2,3,5-tetrahydrocyclopenta[c]chinolin-4-on

2-(Pivaloylamino)phenylboronsäure (F. Guiller, F Nivoliers, A. Godard, F. Marsais, G. Quéguiner, M.A. Siddiqui, V. Snieckus, *J. Org. Chem.* **1995**, *60*, 292) (0.66 g, 3.0 mmol), 3-(Trifluormethylsulfonyloxy)-2,5-dihydrofuran-4-carbonsäure-methylester (0.69 g, 2.5 mmol), Tetrakis(triphenylposphin)palladium (69 mg, 0.06 mmol) und eine 2M wäßrige Na₂CO₃-Lösung (1.26 mL, 2.52 mmol) werden in DME (40 mL) 2.5 h unter Rückfluß erhitzt. Der Ansatz wird mit Essigester verdünnt, mit Wasser und ges. NaCl gewaschen, getrocknet (Na₂SO₄) und i. Vak. eingeengt. Säulenchromatographie an Kieselgel mit Essigester-Hexan liefert 0.65 g (87%) 4-(2-(Pivaloylamino)phenyl)-2,5-dihydrofuran-3-carbonsäure-methylester (Fp. 105 - 6°C). 0.59 g (1.94 mmol) davon werden in konz. Salzsäure 2 h unter Rückfluß erhitzt. Der Ansatz wird auf Eis gegossen und mit Essigester und Dichlormethan extrahiert. Nach Trocknen (Na₂SO₄) und Einengen wird der Rückstand zwei weitere Stunden mit konz. HCl erhitzt und wie beschrieben aufgearbeitet: 0.22 g (58%) Produkt.
¹H-NMR ([D₆]-DMSO): δ = 4.98 (t, 2H), 5.31 (t, 2H), 7.22 (t, 1H), 7.42 (d, 1H), 7.48 (d, 1H), 7.53 (d, 1H), 11.78 (br., 1H).

### 2-Oxa-1,2,3,3a,5,9b-hexahydrocyclopenta[c]chinolin-4-on

Eine Lösung von 2-Oxa-1,2,3,5-tetrahydrocyclopenta[c]chinolin-4-on (0.22 g, 1.17 mmol) in Methanol (24 mL) wird mit Magnesium (0.28 g, 11.7 mmol) und Essigsäure (0.02 mL) versetzt. Nach 6 h wird eine weitereProtion Magnesium (0.28 g, 11.7 mmol) dazugegeben und 12 h bei Raumtemp. gerührt. Der Ansatz wird über ein Glasfilter mit Essigester und Methanol als Eluenten filtriert. Das Filtrat wird eingeengt und säulenchromatographisch (SiO₂) mit Essigester-Methanol gereinigt: 31 mg (14%) Produkt.
¹H-NMR (CDCl₃): δ = 3.26 (ddd, 1H), 3.54 (t, 1H), 3.65 (q, 1H), 4.23 (t, 1H), 4.27 (dd, 1H), 4.43 (dd, 1H), 6.78 (d, 1H), 7.04 (t, 1H), 7.21 (d, 1H), 7.23 (t, 1H), 7.79 (br., 1H).

### 2-Oxa-1,2,3,3a,5,9b-hexahydrocyclopenta[c]chinolin-4-thion

Wie für Beispiel 4 beschrieben, wird 2-Oxa-1,2,3,3a,5,9b-hexahydrocyclopenta[c]chinolin-4-on (53 mg, 0.28 mmol) mit Lawessons Reagenz (121 mg, 0.30 mmol) zu 31 mg (54%) Produkt umgesetzt.
¹H-NMR (CDCl₃): δ = 3.65 (m, 2H), 3.70 (t, 1H), 4.32 (m, 2H), 4.50 (t, 1H), 6.86 (d, 1H), 7.14 (t, 1H), 7.21 - 7.31 (m, 2H), 9.50 (br., 1H).

### 4-Amino-2-oxa-2,3,3a,9b-tetrahydro-1H-cyclopenta[c]chinolin

Wie für Beispiel 4 beschrieben, wird 2-Oxa-1,2,3,3a,5,9b-hexahydrocyclopenta[c]chinolin-4-thion (26 mg, 0.13 mmol) mit 7 N methanolischer Ammoniaklösung (10 mL) zu 23 mg (97%) Produkt umgesetzt. ¹H-NMR ([D₆]-DMSO): δ = 3.31 (m, 1H), 3.58 - 3.75 (m, 3H), 4.18 - 4.26 (m, 2H), 6.97 (t, 1H), 6.99 (d, 1H), 7.14 (t, 1H), 7.18 (d, 1H), 8.40 (br., 2H);
MS (EI) m/e = 188 (M⁺).

### Beispiel 8

### 4-Amino-7-methoxy-2,3,3a,9b-tetrahydro-1H-cyclopenta[c]chinolin

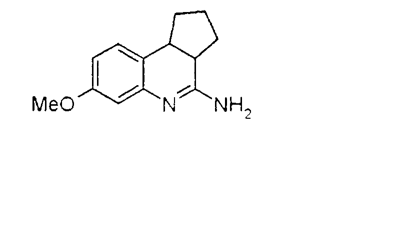

### 7-Methoxy-1,2,3,5-tetrahydrocyclopenta[c]chinolin-4-on

Eine Lösung von 3-Methoxyphenylisocyanat (25.0 g, 168 mmol) in Chloroform (110 mL) wird mit 1-Morpholino-1-cyclopenten (25.3 mL, 168 mmol) versetzt und 15 min unter Rückfluß erhitzt. Der Ansatz wird eingeengt und säulenchromatographisch (SiO₂) mit Essigester-Hexan gereinigt: 34.8 g (87%) Cyclopentanon-2-carbonsäure-(3-methoxyphenyl)amid: ¹H-NMR (CDCl₃): δ =1.80 - 2.55 (m, 6H), 3.18 (t, 1H), 3.81 (s, 3H), 6.68 (dd, 1H), 7.04 (ddd, 1H), 7.22 (t, 1H), 7.33 (t, 1H), 8.76 (br. s, 1H). Das Amid (11.6 g, 50 mmol) wird in konz. Schwefelsäure (40 mL) 30 min bei 100 °C gerührt. Der Ansatz wird auf Eiswasser gegossen, mit Wasser verdünnt und mit Essigester extrahiert. Die vereinigten Extrakte werden getrocknet (Na₂SO₄) und i. Vak. eingeengt: 2.43 g (23%) Produkt.
¹H-NMR (CDCl₃): δ = 2.08 (pent, 2H), 2.73 (t, 2H), 3.06 (t, 2H), 3.81 (s, 3H), 6.82 (dd, 1H), 6.87 (d, 1H), 7.46 (d, 1H), 11.47 (br. s, 1H).

### 7-Methoxy-1,3,3a,5,9b-hexahydrocyclopenta[c]chinolin-4-on

7-Methoxy-1,2,3,5-tetrahydrocyclopenta[c]chinolin-4-on (400 mg, 1.86 mmol) und Magnesium (452 mg, 18.6 mmol) werden analog zu Beispiel 7 in 180 mg (45%) Produkt umgewandelt.
¹H-NMR (CDCl₃): δ = 1.57 - 1.78 (m, 3H), 2.00 - 2.18 (m, 2H), 2.29 (m, 1H), 2.93 (td, 1H), 3.22 (q, 1H), 3.79 (s, 3H), 6.32 (d, 1H), 6.57 (dd, 1H), 7.11 (d, 1H), 8.19 (br. s, 1H).

### 7-Methoxy-1,2,3,3a,5,9b-hexahydrocyclopenta[c]chinolin-4-thion

Analog zu Beispiel 4 wird 7-Methoxy-1,2,3,3a,5,9b-hexahydrocyclopenta[c]chinolin-4-on (180 mg, 0.83 mmol) mit Lawessons Reagenz (370 mg, 0.91 mmol) zu 150 mg (78%) Produkt umgesetzt.
¹H-NMR (CDCl₃): δ = 1.6o - 1.76 (m, 2H), 1.92 (m, 1H), 2.04 - 2.21 (m, 2H), 2.31 (m, 1H), 3.28 (m, 2H), 3.81 (s, 3H), 6.41 (d, 1H), 6.68 (dd, 1H), 7.16 (d, 1H), 9.79 (br. s, 1H).

### 4-Amino-7-methoxy-2,3,3a,9b-tetrahydro-1H-cyclopenta[c]chinolin

Analog zu Beispiel 4 wird 7-Methoxy-1,2,3,3a,5,9b-hexahydrocyclopenta[c]chinolin-4-thion (150 mg, 0.64 mmol) in 7 N methanolischer Ammoniaklösung (25 mL) zu 110 mg (79%) Produkt umgesetzt.
¹H-NMR (CDCl₃): δ = 1.50 - 1.76 (m, 3H), 1.94 (m, 1H), 2.09 (m, 1H), 2.30 (m, 1H), 3.10 - 3.27 (m, 2H), 3.73 (s, 3H), 6 56 (dd, 1H), 6.73 (d, 1H), 6.99 (d, 1H);
MS (EI) m/e = 216 (M⁺).

### Beispiel 9

### 4-Amino-8-methoxy-2,3,3a,9b-tetrahydro-1H-cyclopenta[c]chinolin

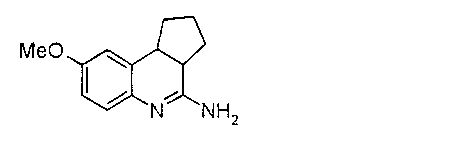

### 8-Methoxy-1,2,3,5-tetrahydrocyclopenta[c]chinolin-4-on

7.10 g Cyclopentanon-2-carbonsäure-(4-methoxyphenyl)amid (G. Jäger, *Chem. Ber.* **1972**, *105,* 137) wird in konz. Schwefelsäure (20 mL) 30 min bei 100 °C gerührt. Der Ansatz wird auf Eiswasser gegossen, mit Wasser verdünnt und mit Essigester extrahiert. Die vereinigten Extrakte werden getrocknet (Na₂SO₄) und i. Vak. eingeengt: 0.78 g (12%) Produkt.
¹H-NMR (CDCl₃): δ = 2.12 (pent. 2H), 2.78 (t, 2H), 3.09 (t, 2H), 3.82 (s, 3H), 7.00 (d, 1H), 7.11 (dd, 1H), 7 29 (d, 1H), 11.42 (br. s, 1H).

### 8-Methoxy-1,2,3,3a,5,9b-hexahydrocyclopenta[c]chinolin-4-on

8-Methoxy-1,2,3,5-tetrahydrocyclopenta[c]chinolin-4-on (0.78g, 3.6 mmol) und Magnesium (2.5 g, 103 mmol) werden analog zu Beispiel 7 zu 80 mg (10%) Produkt umgesetzt.
¹H-NMR (CDCl₃): δ = 1.59 - 1 79 (m, 3H), 2.03 - 2.48 (m, 3H), 2.94 (td, 1H), 3.23 (q, 1H), 3.79 (s, 3H), 6.67 - 6.78 (m, 3H), 8.13 (br. s, 1H).

### 8-Methoxy-1,2,3,3a,5,9b-hexahydrocyclopenta[c]chinolin-4-thion

Analog zu Beispiel 4 wird 8-Methoxy-1,2,3,3a,5,9b-hexahydrocyclopenta[c]chinolin-4-on 80 mg, 0.37 mmol) mit Lawessons Reagenz (404 mg, 1.0 mmol) zu 61 mg (71%) Produkt umgesetzt.
¹H-NMR (CDCl₃): δ = 1.62 - 1.76 (m, 2H) 1 94 (m, 1H), 2.11 - 2.39 (m, 3H), 3.28 (m, 2H), 3.82 (s, 3H), 6.72 (dd, 1H), 6.89 (d, 1H), 6.81 (d, 1H), 9.84 (br. s, 1H).

### 4-Amino-8-methoxy-2,3,3a,9b-tetrahydro-1H-cyclopenta[c]chinolin

Analog zu Beispiel 4 wird 8-Methoxy-1,2,3,3a,5,9b-hexahydrocyclopenta[c]chinolin-4-thion (54 mg, 0.23 mmol) in 7 N methanolischer Ammoniaklösung zu 23 mg (46%) Produkt umgesetzt.
¹H-NMR ([D₆]-DMSO) : δ = 1.44 - 1.62 (m, 3H), 1.87 (m, 1H), 1.98 - 2.15 (m, 2H), 2.66 (q, 1H), 3.19 (q, 1H), 3.69 (s, 3H), 6.62 (dd, 1H), 6.70 (d, 1H), 6.74 (br., 2H), 6.78 (d, 1H);
MS (EI) m/e = 216 (M⁺).

### Beispiel 10

### 4-Amino-7-methyl-2,3,3a,9b-tetrahydro-1H-cyclopenta[c]chinolin

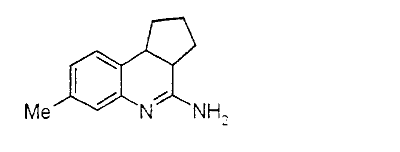

### 7-Methyl-1,2,3,3a,5,9b-hexahydrocyclopenta[c]chinolin-4-on

7-Methyl-1,2,3,5-tetrahydrocyclopenta[c]chinolin-4-on (R.J. Brown, F.W.S. Carver, B.L. Hollingsworth, *J. Chem* Soc. **1961**, 4295) (800 mg, 4.0 mmol) und Magnesium (975 mg, 40 mmol) werden analog zu Beispiel 7 in 500 mg (62%) Produkt umgewandelt.
¹H-NMR (CDCl₃): δ = 1.59 - 1.79 (m, 3H), 2.03 - 2.19 (m, 2H), 2.30 (m, 1H), 2.31 (s, 3H), 2.95 (td, 1H), 3.25 (q, 1H), 6.60 (s, 1H), 6.82 (d, 1H), 7.10 (d, 1H), 8.39 (br. s, 1H).

### 7-Methyl-1,2,3,3a,5,9b-hexahydrocyclopenta[c]chinolin-4-thion

Analog zu Beispiel 4 wird 7-Methyl-1,2,3,3a,5,9b-hexahydrocyclopenta[c]chinolin-4-on (200 mg, 1.0 mmol) mit Lawessons Reagenz (425 mg, 1.1 mmol) zu 230 mg Produkt umgesetzt.
¹H-NMR (CDCl₃): δ = 1.60 - 1.75 (m, 2H), 1.93 (m, 1H), 2.07 - 2.34 (m, 3H), 2.32 (s, 3H), 3.29 (m, 2H), 6.68 (s, 1H), 6.93 (d, 1H), 7.14 (d, 1H), 9.98 (br. s, 1H).

### 4-Amino-7-methyl-2,3,3a,9b-tetrahydro-1H-cyclopenta[c]chinolin

In Anlehnung an Beispiel 4 wird 7-Methyl-1,2,3,3a,5,9b-hexahydrocyclopenta[c]chinolin-4-thion (230 mg, 1.05 mmol) in 7 N methanolischer Ammoniaklösung (25 mL) zu 210 mg (99%) Produkt umgesetzt. ¹H-NMR (CDCl₃): δ = 1.51 - 1.78 (m, 3H), 1.94 (m, 1H), 2.03 - 2.33 (m, 2H), 2.23 (s, 3H), 3.05 (q, 1H), 3.24 (q, 1H), 6.17 (br., 2H), 6.81 (d, 1H), 6.93 (s, 1H), 7.00 (d, 1H);
MS (EI) m/e = 200 (M⁺).

### Beispiel 11

### 4-Amino-8-methyl-2,3,3a,9b-tetrahydro-1H-cyclopenta[c]chinolin

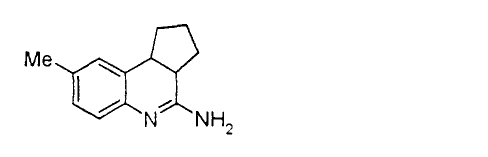

### 8-Methyl-1,2,3,3a,5,9b-hexahydrocyclopenta[c]chinolin-4-on

8-Methyl-1,2,3,5-tetrahydrocyclopenta[c]chinolin-4-on (R.J. Brown, F.W.S. Carver, B.L. Hollingsworth, *J. Chem Soc.* **1961**, 4295) (1.20 g, 6.0 mmol) und Magnesium (1.46 g, 60 mmol) werden analog zu Beispiel 7 in 0.48 g (40%) Produkt umgewandelt.
¹H-NMR (CDCl₃): δ = 1.48 - 1.80 (m, 3H), 2.03 - 2.18 (m, 2H), 2.29 (s, 3H), 2.33 (m, 1H), 2.94 (td, 1H), 3.23 (q, 1H), 6.68 (d, 1H), 6.97 (d, 1H), 7.02 (s, 1H), 8.44 (br. s, 1H).

### 8-Methyl-1,2,3,3a,5,9b-hexahydrocyclopenta[c]chinolin-4-thion

Analog zu Beispiel 4 wird 8-Methyl-1,2,3,3a,5,9b-hexahydrocyclopenta[c]chinolin-4-on (200 mg, 1.0 mmol) mit Lawessons Reagenz (425 mg, 1.1 mmol) zu 210 mg (97%) Produkt umgesetzt.
¹H-NMR (CDCl₃): δ = 1.62 - 1.75 (m, 2H), 1.91 (m, 1H), 2.07 - 2.38 (m, 3H), 2.31 (s, 3H), 3.28 (m, 2H), 6.75 (d, 1H), 6.99 (d, 1H), 7.06 (s, 1H), 9.87 (br. s, 1H).

### 4-Amino-8-methyl-2,3,3a,9b-tetrahydro-1H-cyclopenta[c]chinolin

In Anlehnung an Beispiel 4 wird 8-Methyl-1,2,3,3a,5,9b-hexahydrocyclopenta[c]chinolin-4-thion (200 mg, 0.92 mmol) in 7 N methanolischer Ammoniaklösung (20 mL) zu 190 mg (99%) Produkt umgesetzt. ¹H-NMR (CDCl₃): δ = 1.52 - 1.78 (m, 3H), 1 91 (m, 1H), 2.07 - 2.35 (m, 2H), 2.25 (s, 3H), 3.11 (q, 1H), 3.22 (q, 1H), 6.27 (br., 2H), 6.87 (s, 1H), 6.88 (d, 1H), 7.02 (d, 1H);
MS (EI) m/e = 200 (M⁺).

### Beispiel 12

### 4-Amino-8-chlor-2,3,3a,9b-tetrahydro-1H-cyclopenta[c]chinolin

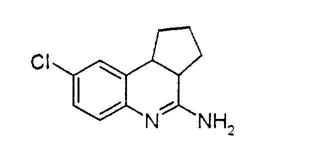

### 8-Chlor-1,2,3,3a,5,9b-hexahydrocyclopenta[c]chinolin-4-on

Eine Lösung von 1,2,3,3a,5,9b-Hexahydrocyclopenta[c]chinolin-4-on (1.12 g, 6.0 mmol) und *N*-Chlorsuccinimid (0.80 g, 6.0 mmol) in DMF (60 mL) wird 48 h auf 100 °C erwärmt, auf Eiswasser gegossen und mit Essigester extrahiert. Die vereinigten Extrakte werden mit 10 proz. Schwefelsäure und Wasser gewaschen, getrocknet (Na₂SO₄) und i. Vak. eingeengt. Der Rückstand wird an Kieselgel mit Essigester-Hexan gereinigt: 0.55 g (84%) Produkt.
¹H-NMR (CDCl₃) : δ = 1.60 - 1.82 (m, 3H), 2.05 - 2.22 (m, 2H), 2.32 (m, 1H), 2.95 (td, 1H), 3.25 (q, 1H), 6.76 (d, 1H), 7.13 (dd, 1H), 7.19 (d, 1H), 8.89 (br. s, 1H).

### 8-Chlor-4-methoxy-2,3,3a,9b-tetrahydro-1H-cyclopenta[c]chinolin

Eine Lösung von 8-Chlor-1,2,3,3a,5,9b-hexahydrocyclopenta[c]chinolin-4-on (200 mg, 0.9 mmol) in Dichlormethan (20 mL) wird mit Trimethyloxoniumtetrafluoroborat (134 mg, 0.9 mmol) 24 h bei Raumtemp. gerührt. Nach Zugabe von Triethylamin (0.13 mL, 0.9 mmol) wird der Ansatz eingeengt und säulenchromatographisch (SiO₂, 40 proz. feucht) mit Essigester-Hexan gereinigt: 180 mg (85%) Produkt.
¹H-NMR (CDCl₃): δ = 1.57 - 1.78 (m, 3H), 1.93 (m, 1H), 2.07 - 2.23 (m, 2H), 2.84 (ddd, 1H), 3.28 (q, 1H), 3.88 (s, 3H), 7.07 - 7.18 (m, 3H).

### 4-Amino-8-chlor-2,3,3a,9b-tetrahydro-1H-cyclopenta[c]chinolin

Eine Suspension vom 8-Chlor-4-methoxy-2,3,3a,9b-tetrahydro-1H-cyclopenta[c]chinolin (180 mg, 0.76 mmol) und Ammoniumchlorid (200 mg, 3.81 mmol) in Ethanol (15 mL) wird 8 h unter Rückfluß erhitzt und 3 Tage bei Raumtemp. belassen. Das Lösungsmittel wird abdestilliert und der Rückstand an Kieselgel mit Dichlormethan-Methanol gereinigt: 50 mg (30%) Produkt.
¹H-NMR ([D₆]-DMSO): δ = 1.48 - 1.77 (m, 3H), 2.02 (m, 1H), 2.13 - 2.28 (m, 2H), 3.21 (q, 1H), 3.47 (q, 1H), 7.16 (d, 1H), 7.33 (dd, 1H), 7.44 (d, 1H), 9.63 (br., 2H);
MS (EI) m/e = 220 (M⁺).

### Beispiel 13

### 4-Amino-6,8-dichlor-2,3,3a,9b-tetrahydro-1H-cyclopenta[c]chinolin

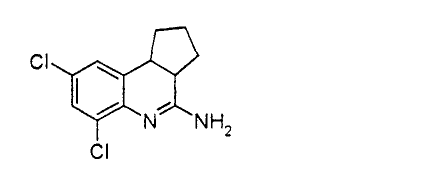

### 6,8-Dichlor-1,2,3,3a,5,9b-hexahydrocyclopenta[c]chinolin-4-on

Eine Lösung von 1,2,3,3a,5,9b-Hexahydrocyclopenta[c]chinolin-4-on (300 mg, 1.6 mmol) in DMF (20 mL) wird mit *N*-Chlorsuccinimid (641 mg, 4.8 mmol) versetzt und 24 h bei 100 °C gerührt. Der Ansatz wird auf Eiswasser gegossen und mit Essigester extrahiert. Die vereinigten Extrakte werden mit 10 proz. Schwefelsäure und Wasser gewaschen, getrocknet (Na₂SO₄) und i. Vak. eingeengt. Der Rückstand wird an Kieselgel mit Essigester-Hexan gereinigt: 350 mg (85%) Produkt.
¹H-NMR (CDCl₃): δ = 1.55 - 1.84 (m, 3H), 2.03 - 2.19 (m, 2H), 2.38 (m, 1H), 2.97 (td, 1H), 3.25 (q, 1H), 7.13 (d, 1H), 7.25 (d, 1H), 7.77 (br. s, 1H).

### 6,8-Dichlor-1,2,3,3a,5,9b-hexahydrocyclopenta[c]chinolin-4-thion

6,8-Dichlor-1,2,3,3a,5,9b-hexahydrocyclopenta[c]chinolin-4-on (100 mg, 0.39 mmol) wird mit Lawessons Reagenz (412 mg, 1.02 mmol) 2 h in THF (20 mL) erhitzt. Der Ansatz wird eingeengt und säulenchromatographisch (SiO₂) mit Essigester-Hexan gereinigt: 100 mg (94%) Produkt.
¹H-NMR (CDCl₃): δ = 1.67 - 1.79 (m, 2H), 1.85 (m, 1H), 2.16 (m, 1H), 2.25 - 2.38 (m, 2H), 3.29 (m, 2H), 7.16 (d, 1H), 7.27 (d, 1H), 9.57 (br. s, 1H).

### 4-Amino-6,8-dichlor-2,3,3a,9b-tetrahydro-1H-cyclopenta[c]chinolin

Analog zu Beispiel 4 wird 6,8-Dichlor-1,2,3,3a,5,9b-hexahydrocyclopenta[c]chinolin-4-thion (100 mg, 0.37 mmol) mit 7 N methanolischer Ammoniaklösung (20 mL) zu 70 mg (74%) Produkt umgesetzt. ¹H-NMR ([D₆]-DMSO): δ = 1.46 - 1.68 (m, 3H), 1.82 (m, 1H), 2.00 - 2.16 (m, 2H), 2.69 (q, 1H), 3.23 (q, 1H), 6.88 (br., 2H), 7 11 (d, 1H), 7.21 (d, 1H);
MS (EI) m/e = 254 (M⁺).

### Beispiel 14

### 4-Amino-8-brom-2,3,3a,9b-tetrahydro-1H-cyclopenta[c]chinolin

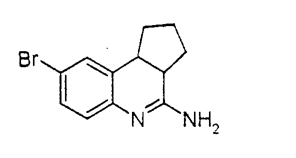

### 8-Brom-1,2,3,3a,5,9b-hexahydrocyclopenta[c]chinolin-4-on

Eine Lösung von 1,2,3,3a,5,9b-Hexahydrocyclopenta[c]chinolin-4-on (374 mg, 2.0 mmol) in DMF (10 mL) wird mit *N*-Bromsuccinimid (356 mg, 2.0 mmol) versetzt. Nach 15 min Rühren bei Raumtemp. wird der Ansatz 15 min auf 100 °C erwärmt, mit Wasser verdünnt und mit Methyl-*tert*-butylether extrahiert. Die vereinigten Extrakte werden getrocknet (Na₂SO₄) und i. Vak. eingeengt. Der Rückstand wird an Kieselgel mit Essigester-Hexan gereinigt: 470 mg (88%) Produkt.
Fp. 170 - 2 °C.

### 8-Brom-1,2,3,3a,5,9b-hexahydrocyclopenta[c]chinolin-4-thion

8-Brom-1,2,3,3a,5,9b-hexahydrocyclopenta[c]chinolin-4-on (100 mg, 0.38 mmol) wird mit Lawessons Reagenz (404 mg, 10 mmol) 5 h in THF (20 mL) erhitzt. Der Ansatz wird eingeengt und saufenchromatographisch (SiO₂) mit Essigester-Hexan gereinigt: 82 mg (76%) Produkt
¹H-NMR (CDCl₃-[D₆]-DMSO): δ = 1.49 (m, 2H), 1.72 (m, 1H), 1.90 - 2.16 (m, 3H), 3.05 (m, 2H), 6.83 (d, 1H), 7 07 (dd 1H), 7.16 (d, 1H), 11.58 (br. s, 1H).

### 4-Amino-8-brom-2,3,3a,9b-tetrahydro-1H-cyclopenta[c]chinolin

Analog zu Beispiel 4 wird 8-Brom-1,2,3,3a,5,9b-hexahydrocyclopenta[c]chinolin-4-thion (76 mg, 0.30 mmol) mit 7 N methanolischer Ammoniaklösung (20 mL) zu 67 mg (84%) Produkt umgesetzt.
Fp. 171 - 3 °C;
MS (EI) m/e = 264/266 (M⁺).

### Beispiel 15

### 4-Amino-8-(trifluormethyl)-2,3,3a,9b-tetrahydro-1H-cyclopenta[c]chinolin

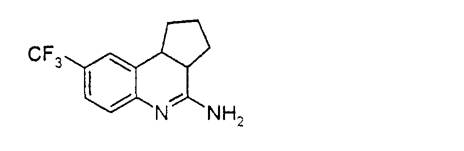

### Pivalinsäure-(4-trifluormethyl)anilid

Eine Lösung von 4-(Trifluormethyl)anilin (6.3 mL, 50 mmol) in Dichlormethan (200 mL) wird mit 4-(Dimethylamino)pyridin (30 mg), Pyridin (12 mL, 150 mmol) und vorsichtig mit Pivaloylchlorid (9.2 mL, 75 mmol) versetzt. Nach Abklingen der exothermen Reaktion wird noch 4 h bei Raumtemp. gerührt, bevor Wasser (50 mL) dazugegeben wird. Nach 45 min wird der Ansatz mit Methyl-*tert*-butylether (300 mL) verdünnt, mit 10 proz. Schwefelsäure (2 x 100 mL) gewaschen, getrocknet (Na₂SO₄) und i. Vak. eingeengt. Der Rückstand wird aus Hexan umkristallisiert: 12.09 g (99%) Produkt.
Fp. 157 - 8 °C.

### 1-(Trifluormethylsulfonyloxy)cyclopent-1-en-2-carbonsäure-ethylester

Eine Lösung von Cyclopentanon-2-carbonsäure-ethylester (7.2 mL, 50 mmol) in Dichlormethan (200 mL) wird bei -70 °C mit Triethylamin (7.6 mL, 55 mmol) versetzt. Nach 10 min wird Trifluormethansulfonsäureanhydrid (8.2 mL, 50 mmol) langsam dazugetropft. Der Ansatz wird 5 h unter Erwärmung auf Raumtemp.gerührt, mit Methyl-*tert*-butylether verdünnt, mit Wasser und ges. NaCl gewaschen, getrocknet (Na₂SO₄) und i. Vak. eingeengt. Der Rückstand wird säulenchromatographisch (SiO₂) mit Essigester-Hexan gereinigt: 12.53 g (87%) Produkt.
¹H-NMR (CDCl₃): δ = 1.33 (t, 3H), 2.03 (pent, 1H), 2.74 (m, 4H), 4.28 (q, 2H).

### 8-(Trifluormethyl)-1,2,3,5-tetrahydrocyclopenta[c]chinolin-4-on

Eine Lösung von Pivalinsäure-(4-trifluormethyl)anilid (3.68 g, 15 mmol) in THF (50 mL) wird bei 0 °C mit 1.6 M n-BuLi in Hexan (28.2 mL, 45 mmol) behandelt. Nach 4 h Rühren bei 0 °C wird Borsäure-trimethylorthoester (5.1 mL, 45 mmol) dazugegeben und 2 h bei 0 °C gerührt. Der Ansatz wird mit Wasser verdünnt, mt 10 proz. Schwefelsäure angesäuert und mit Essigester extrahiert. Die vereinigten Extrakte werden getrocknet und i. Vak. eingeengt. Der Rückstand (4.01 g) wird mit 1-(Trifluormethylsulfonyloxy)cyclopent-1-en-2-carbonsäureethylester (2.3 g, 7.8 mmol), Tetrakis(triphenylphosphin)palladium (0.2 g, 0.18 mmol) und 1 M wäßriger Na₂CO₃ (7.8 mL, 7.8 mmol) in 1,2-Dimethoxyethan (60 mL) 4 h unter Rückfluß erhitzt. Das Reaktionsgemisch wird mit Methyl-*tert*-butylether (300 mL) verdünnt, mit Wasser und ges. NaCl gewaschen, getrocknet (Na₂SO₄) und i. Vak. eingeengt. Säulenchromatographie an Kieselgel mit Methyl-*tert*-butylether-Hexan liefert 4.7 g (82%) 2-[2-(Pivaloylamino)-5-(trifluormethyl)phenyl]-cyclopent-1-en-1-carbonsäureethylester (ausgewählte ¹H-NMR-Daten (CDCl₃): δ = 1.06 (t, 3H), 2.07 (pent 2H), 2.79 (t, 2H), 2.90 (t, 2H), 4.05 (q, 2H)). Davon werden 2.0 g (5.2 mmol) in konz. Salzsäure 6 h unter Rückfluß erhitzt. Der Ansatz wird auf Eiswasser gegossen und filtriert. Der Filterrückstand wird mit Wasser gewaschen: 1.08 g (82%) Produkt.
¹H-NMR ([D₆]-DMSO): δ = 2.14 (pent, 2H), 2.81 (t, 2H), 3.16 (t, 2H). 7.52 (d, 1H), 7.78 (d, 1H), 7.87 (s, 1H), 11.89 (br. s, 1H).

### 8-(Trifluormethyl)-1,2,3,3a,5,9b-hexahydrocyclopenta[c]chinolin-4-on

Analog zu Beispiel 7 wird 8-(Trifluormethyl)-1,2,3,5-tetrahydrocyclopenta[c]chinolin-4-on (1.08 g, 4.3 mmol) mit Magnesium (2.24 g, 92 mmol) zu 0.55 g (50%) Produkt umgesetzt.
¹H-NMR (CDCl₃): δ = 1.58 - 1.81 (m, 3H), 2.06 - 2.22 (m, 2H), 2.32 (m, 1H), 2.99 (td, 1H), 3.31 (q, 1H), 6.84 (d, 1H), 7.42 (d, 1H), 7.47 (s, 1H), 8.87 (br. s, 1H).

### 8-(Trifluormethyl)-1,2,3,3a,5,9b-hexahydrocyclopenta[c]chinolin-4-thion

Analog zu Beispiel 4 wird 8-(Trifluormethyl)-1,2,3,3a,5,9b-hexahydrocyclopenta[c]chinolin-4-on (0.15 g, 0.6 mmol) mit Lawessons Reagenz (0.49 g, 1.2 mmol) zu 0.10 g (61%) Produkt umgesetzt.
¹H-NMR (CDCl₃): δ = 1.66 - 1.81 (m, 2H), 1.92 (m, 1H), 2.13 - 2.42 (m, 3H), 3.33 (m, 2H), 6.92 (d, 1H), 7.47 (d, 1H), 7.53 (s, 1H), 9.73 (br. s, 1H).

### 4-Amino-8-(trifluormethyl)-2,3,3a,9b-tetrahydro-1H-cyclopenta[c]chinolin

Analog zu Beispiel 4 wird 8-(Trifluormethyl)-1,2,3,3a,5,9b-hexahydrocyclopenta[c]chinolin-4-thion (93 mg, 0.34 mmol) in 7 N methanolischer Ammoniaklösung (20 mL) zu 66 mg (76%) Produkt umgesetzt. Fp. 163 - 5 °C;
¹H-NMR (CDCl₃): δ = 1.63 - 2.01 (m, 4H), 2.22 (m, 2H), 2.81 (q, 1H), 3.35 (q, 1H), 5.12 (br, 2H), 7.07 (d, 1H), 7 37 (s, 1H), 7.39 (d, 1H);
MS (EI) m/e = 254 (M⁺).

### Beispiel 16

### 4-Amino-8-cyano-2,3,3a,9b-tetrahydro-1H-cyclopent[a]chinolin

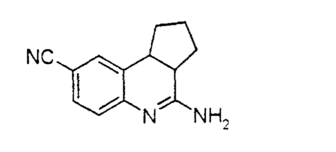

### 8-Cyano-1,2,3,3a,5,9b-hexahydrocyclopenta[c]chinolin-4-on

Eine Lösung von 8-Brom-1,2,3,3a,5,9b-hexahydrocyclopenta[c]chinolin-4-on (230 mg, 0.86 mmol) in DMF (20 mL) wird mit Kupfer(I)cyanid (77 mg, 0.86 mmol) versetzt und 16 h unter Rückfluß erhitzt. Der Ansatz wird mit Dichlormethan verdünnt, mit ges. NH₄Cl gewaschen, getrocknet (Na₂SO₄) und i. Vak. eingeengt. Reinigung an Kieselgel mit Essigester-Hexan liefert 70 mg (38%) Produkt.
¹H-NMR (CDCl₃): δ = 1.57 - 1.82 (m, 3H), 2.09 - 2.25 (m, 2H), 2.34 (m, 1H), 3.01 (td, 1H), 3.30 (q, 1H), 6.89 (d, 1H), 7.48 (d, 1H), 7.50 (s, 1H), 9.14 (br. s, 1H).

### 8-Cyano-1,2,3,3a,5,9b-hexahydrocyclopenta[c]chinolin-4-thion

8-Cyano-1,2,3,3a,5,9b-hexahydrocyclopenta[c]chinolin-4-on (70 mg, 0.33 mmol) wird mit Lawessons Reagenz (146 mg, 0.36 mmol) 2 h in DME (20 mL) auf 80 °C erhitzt. Der Ansatz wird eingeengt und säulenchromatographisch (SiO₂) mit Essigester-Hexan gereinigt: 50 mg (66%) Produkt.
¹H-NMR (CDCl₃): δ = 1.64 - 1.79 (m, 2H), 1.88 (m, 1H), 2.13 - 2.42 (m, 3H), 3.33 (m, 2H), 6.92 (d, 1H), 7.52 (d, 1H), 7.57 (s, 1H), 9.80 (br. s, 1H).

### 4-Amino-8-cyano-2,3,3a,9b-tetrahydro-1H-cyclopenta[c]chinolin

Analog zu Beispiel 4 wird 8-Cyano-1,2,3,3a,5,9b-hexahydrocyclopenta[c]chinolin-4-thion (40 mg, 0.18 mmol) mit 7 N methanolischer Ammoniaklösung (10 mL) zu 30 mg (81%) Produkt umgesetzt. ¹H-NMR ([D₆]-DMSO): δ = 1.43 - 1.67 (m, 3H), 1.89 (m, 1H), 2.01 - 2.19 (m, 2H), 2.73 (q, 1H), 3.26 (q, 1H), 6.89 (d, 1H), 7.20 (br., 2H), 7.42 (d, 1H), 7.52 (s, 1H);
MS (EI) m/e = 211 (M⁺).

### Beispiel 17

### 4-Amino-8-fluor-2,3,3a,9b-tetrahydro-1H-cyclopenta[c]chinolin

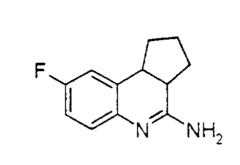

### 8-Fluor-1,2,3,5-tetrahydrocyclopenta[c]chinolin-4-on

Eine Lösung von 4-Fluorphenylisocyanat (5.0 g, 36.5 mmol) in Chloroform (25 mL) wird mit 1-Morpholino-1-cyclopenten (5.5 mL, 36.5 mmol) versetzt und 15 min unter Rückfluß erhitzt. Der Ansatz wird eingeengt und säulenchromatographisch (SiO₂) mit Essigester-Hexan gereinigt: 7.6 g (94%) Cyclopentanon-2-carbonsäure-(4-fluorphenyl)amid, 1H-NMR (DSC842). Das Amid wird in konz. Schwefelsäure (30 mL) 30 min bei 100 °C gerührt. Der Ansatz wird auf Eiswasser gegossen und filtriert. Der Filterrückstand wird aus Ethanol umkristallisiert: 2.02 g (29%) Produkt.
Fp. 287 °C.

### 8-Fluor-1,2,3,3a,5,9b-hexahydrocyclopenta[c]chinolin-4-on

8-Fluor-1,2,3,5-tetrahydrocyclopenta[c]chinolin-4-on (1.0 g, 4.9 mmol) und Magnesium (1.2 g, 49.2 mmol) werden analog zu Beispiel 7 zu 280 mg (28%) Produkt umgesetzt.
¹H-NMR (CDCl₃): δ = 1.60 - 1.80 (m, 3H), 2.05 - 2.20 (m, 2H), 2.31 (m, 1H), 2.94 (td, 1H), 3.25 (q, 1H), 6.75 (dd, 1H), 6.87 (td, 1H), 6.93 (dd, 1H), 8.69 (br. s, 1H).

### 8-Fluor-1,2,3,3a,5,9b-hexahydrocyclopenta[c]chinolin-4-thion

Analog zu Beispiel 4 wird 8-Fluor-1,2,3,3a,5,9b-hexahydrocyclopenta[c]chinolin-4-on (280 mg, 1.36 mmol) mit Lawessons Reagenz (608 mg, 1.5 mmol) zu 240 mg (80%) Produkt umgesetzt.
¹H-NMR (CDCl₃): δ = 1.59 - 1.77 (m, 2H), 1.90 (m, 1H), 2.10 - 2.25 (m, 2H), 2.32 (m, 1H), 3.29 (m, 2H), 6.82 - 6.92 (m, 2H), 6.97 (dd, 1H), 10.08 (br. s, 1H).

### 4-Amino-8-fluor-2,3,3a,9b-tetrahydro-1H-cyclopenta[c]chinolin

Analog zu Beispiel 4 wird 8-Fluor-1 ,2,3,3a,5,9b-hexahydrocyclopenta[c]chinolin-4-thion (240 mg, 1.08 mmol) in 7 N methanolischer Ammoniaklösung (30 mL) zu 210 mg (95%) Produkt umgesetzt.
¹H-NMR ([D₆]-DMSO): δ = 1.47 - 1.70 (m, 3H), 1.94 (m, 1H), 2.07 - 2.21 (m, 2H), 2.94 (q, 1H), 3.30 (br., 2H), 3.36 (q, 1H), 6.92 - 7.01 (m, 2H), 7.11 (dd, 1H);
MS (EI) m/e = 204 (M⁺).

### Beispiel 18

### 4-Amino-7-fluor-2,3,3a,9b-tetrahydro-1H-cyclopenta[c]chinolin

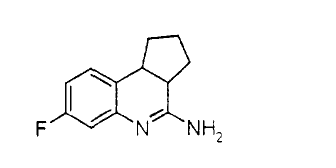

### 7-Fluor-1,2,3,5-tetrahydrocyclopenta[c]chinolin-4-on

Analog Beispiel 17 wird aus 3-Fluorphenylisocyanat (5.0 g, 36.5 mmol) und 1-Morpholino-1-cyclopenten (5.5 mL, 36.5 mmol) Cyclopentanon-2-carbonsäure-(3-fluorphenyl)amid (6.75 g, 84%) ¹H-NMR (DSC841) hergestellt. Das Amid wird in konz. Schwefelsäure (30 mL : 30 min bei 100 °C gerührt. Der Ansatz wird auf Eiswasser gegossen und filtriert. Der Filterrückstand wird aus Ethanol umkristallisiert: 5.14 g (83%) Produkt.
Fp. 283 °C.

### 7-Fluor-1,2,3,3a,5,9b-hexahydrocyclopenta[c]chinolin-4-on

7-Fluor-1,2,3,5-tetrahydrocyclopenta[c]chinolin-4-on (1.0 g, 4.9 mmol) und Magnesium (1.2 g, 49.2 mmol) werden analog zu Beispiel 7 zu 170 mg (17%) Produkt umgesetzt.
¹H-NMR (CDCl₃): δ = 1.56 - 1.82 (m, 3H, 2.02 - 2.19 (m, 2H), 2.32 (m, 1H), 2.96 (td, 1H), 3.24 (q, 1H), 6.53 (dd. 1H ; 6.70 (td, 1H), 7.14 (dd, 1H), 8.60 (br. s, 1H).

### 7-Fluor-1,2,3,3a,5,9b-hexahydrocyclopenta[c]chinolin-4-thion

Analog zu Beispiel 4 wird 7-Fluor-1,2,3,3a,5,9b-hexahydrocyclopenta[c]chinolin-4-on (170 mg, 0.83 mmol) mit Lawessons Reagenz (370 mg, 0.91 mmol) zu 170 mg (93%) Produkt umgesetzt.
¹H-NMR (CDl₃): δ = 1.63 - 1 77 (m, 2H), 1 89 (m, 1H), 2.08 - 2.40 (m, 3H), 3.28 (m, 2H), 6.59 (dd, 1H), 6.82 (td, 1H), 7.21 (dd, 1H), 9.76 (br. s, 1H)

### 4-Amino-7-fluor-2,3,3a,9b-tetrahydro-1H-cyclopenta[c]chinolin

Analog zu Beispiel 4 wird 7-Fluor-1,2,3,3a,5,9b-hexahydrocyclopenta[c]chinolin-4-thion (170 mg, 0.77 mmol) in 7 N methanolischer Ammoniaklösung (20 mL) zu 160 mg (99%) Produkt umgesetzt. ¹H-NMR ([D₆]-DMSO): δ = 1.43 - 1.72 (m, 3H), 1.90 (m, 1H), 2.07 - 2.21 (m, 2H), 2.96 (q, 1H), 3.30 (br., 2H), 3.32 (q, 1H), 6.76 (m, 1H), 6.81 (m, 1H), 7.24 (t, 1H);
MS (EI) m/e = 204 (M⁺).

### Beispiel 19

### 4-Amino-6-fluor-2,3,3a,9b-tetrahydro-1H-cyclopenta[c]chinolin

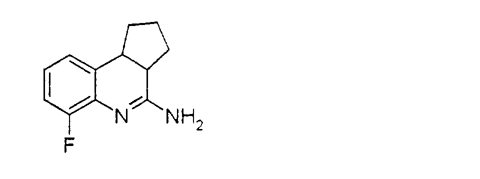

### Pivalinsäure-2-fluoranilid

Eine Lösung von 2-Fluoranilin (4.8 mL, 50 mmol) in Dichlormethan (200 mL) wird mit Pyridin (12 mL, 150 mmol) und vorsichtig mit Pivaloylchlorid (9.2 mL, 75 mmol) versetzt. Nach Abklingen der exothermen Reaktion wird der Ansatz noch 1 h bei Raumtemp. gerührt, mit Methyl-*tert*-butylether verdünnt, mit 10 proz. Salzsäure und ges. NaCl gewaschen gewaschen, getrocknet (Na₂SO₄) und i. Vak. eingeengt. Der Rückstand wird aus Hexan umkristallisiert: 4.86 g (50%) Produkt.
Fp. 77 - 8 °C.

### 6-Fluor-1,2,3,5-tetrahydrocyclopenta[c]chinolin-4-on

Eine Lösung von Pivalinsäure-2-fluoranilid (3.68 g, 15 mmol) in THF (50 mL) wird bei 0 °C mit 1.6 M *n*-BuLi in Hexan (28.2 mL, 45 mmol) behandelt. Nach 2 h Rühren bei 0 °C wird Borsäure-trimethylorthoester (5.1 mL, 45 mmol) dazugegeben und 2 h bei 0 °C gerührt. Der Ansatz wird mit Wasser verdünnt, mit 10 proz. Schwefelsäure angesäuert (pH 3) und mit Essigester extrahiert. Die vereinigten Extrakte werden getrocknet und i. Vak. eingeengt. Der Rückstand wird mit 1-(Trifluormethylsulfonyloxy)cyclopent-1-en-2-carbonsäure-ethylester (3.75 g, 13.0 mmol), Tetrakis(triphenylphosphin)palladium (0 35 g, 0.3 mmol) und 1 M wäßriger Na₂CO₃ (13 0 mL, 13.0 mmol) in 1,2-Dimethoxyethan (100 mL) 4 h unter Rückfluß erhitzt. Das Reaktionsgemisch wird mit Methyl-*tert*-butylether (300 mL) verdünnt, mit Wasser und ges. NaCl gewaschen, getrocknet (Na₂SO₄) und i. Vak. eingeengt. Säulenchromatographie an Kieselgel mit Methyl-*tert*-butylether-Hexan liefert 4.7 g (82%) 2-[2-(Pivaloylamino)-3-fluorphenyl]-cyclopent-1-en-1-carbonsäure-ethylester, ¹H-NMR (CDCl₃): δ = 1.21 (t, 3H), 1.26 (s, 9H), 1.98 (pent, 2H), 2.80 (m, 4H), 4.12 (q, 2H), 6.93 (d, 1H), 7.07 (t, 1H), 7.20 (td, 1H), 7.58 (br. s, 1H). Davon wird 1.0 g (3.0 mmol) in konz. Salzsäure 6 h unter Rückfluß erhitzt. Der Ansatz wird mit Wasser verdünnt und filtriert: 494 mg (80%) Produkt.
¹H-NMR (CDCl₃): δ = 2.23 (pent, 2H), 3.01 (t, 2H), 3.14 (t, 2H), 7.10 - 7.33 (m, 3H), 9.14 (br. s, 1H).

### 6-Fluor-1,2,3,3a,5,9b-hexahydrocyclopenta[c]chinolin-4-on

Analog zu Beispiel 7 wird 6-Fluor-1 ,2,3,5-tetrahydrocyclopenta[c]chinolin-4-on (494 mg, 2.4 mmol) mit Magnesium (1.22 g, 50.0 mmol) zu 280 mg (56%) Produkt umgesetzt.
¹H-NMR (CDCl₃): δ = 1.58 - 1.81 (m, 3H), 2.12 (m, 2H), 2.32 (m, 1H), 2.98 (td, 1H), 3.31 (q, 1H), 6.91 - 7.07 (m, 3H), 7.67 (br. s, 1H).

### 6-Fluor-1,2,3,3a,5,9b-hexahydrocyclopenta[c]chinolin-4-thion

Eine Lösung von 6-Fluor-1,2,3,3a,5,9b-hexahydrocyclopenta[c]chinolin-4-on (100 mg, 0.48 mmol) und Lawessons Reagenz (512 mg, 1.26 mmol) in THF (20 mL) wird 1.5 h unter Rückfluß erhitzt und eingeengt. Säulenchromatographie des Rückstands mit Essigester-Hexan liefert 100 mg (93%) Produkt.
¹H-NMR (CDCl₃): δ = 1.62 - 1.80 (m, 2H), 1.93 (m, 1H), 2.12 - 2.40 (m, 3H), 3.32 (m, 2H), 6.92 - 7.06 (m, 3H), 9.46 (br. s, 1H).

### 4-Amino-6-fluor-2,3,3a,9b-tetrahydro-1H-cyclopenta[c]chinolin

Analog zu Beispiel 4 wird 6-Fluor-1,2,3,3a,5,9b-hexahydrocyclopenta[c]chinolin-4-thion (100 mg, 0.45 mmol) in 7 N methanolischer Ammoniaklösung (15 mL) zu 59 mg (64%) Produkt umgesetzt.
¹H-NMR ([D₆]-DMSO): δ = 1.45 - 1.71 (m, 3H), 1.87 (m, 1H), 2.01 - 2.17 (m, 2H), 2.68 (q, 1H), 3.22 (q, 1H), 6.53 - 6.97 (m, 5H);
MS (EI) m/e = 204 (M⁺).

### Beispiel 20

### 4-Amino-7-(2-furanyl)-2,3,3a,9b-tetrahydro-1H-cyclopenta[c]chinolin

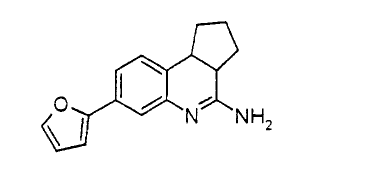

### 7-Brom-1,2,3,5-tetrahydrocyclopenta[c]chinolin-4-on

Zu einer Lösung von 3-Bromphenylisocyanat (20.0 g, 101 mmol) in Chloroform (100 mL) wird vorsichtig 1-Morpholino-1-cyclopenten (15.2 mL, 101 mmol) getropft. Der Ansatz wird 15 min unter Rückfluß erhitzt und i. Vak. eingeengt. Säulenchromatographie an Kieselgel mit Hexan - Essigester lieferte 25.0 g (88.6 mmol) Cyclopentan-2-on-1-carbonsäure-(3-bromphenyl)amid. Diese werden mit werden mit konz. Schwefelsäure (83 mL) versetzt und 30 min bei 90 °C gerührt. Nach Abkühlen auf Raumtemp. wird der Ansatz auf 600 g Eis gegossen, der ausgefallene Feststoff abgesaugt und aus Ethanol umkristallisiert: 17.0 g Produkt.
¹H-NMR ([D₆]DMSO): δ = 2.11 (pent, 2H), 2.74 (t, 2H), 3.07 (t, 2H), 7.34 (dd, 1H), 7.49 (d, 1H), 7.52 (d, 1H), 11.68 (s, 1H).

### 7-(2-Furanyl)-1,2,3,5-tetrahydrocyclopenta[c]chinolin-4-on

Eine Suspension von 7-Brom-1,2,3,5-tetrahydrocyclopenta[c]chinolin-4-on (1.32 g, 5.0 mmol) in Toluol (200 mL) wird mit 2-(Tributylstannyl)furan (1.7 ml, 5.5 mmol) und Tetrakis(triphenylphosphin)palladium (0.29 g, 0.25 mmol) versetzt. Das Reaktionsgemisch wird entgast, mit Stickstoff belüftet, 15 h bei Raumtemp. gerührt und 4.5 h auf 110°C erhitzt. Der Ansatz wird mit Kieselgel versetzt und i. Vak. eingeengt. Säulenchromatographie des Rückstands an Kieselgel mit Hexan-Essigester liefert 1.33 g Produkt.
¹H-NMR ([D₆]DMSO/CDCl₃): δ = 2.00 (pent, 2H), 2.70 (t, 2H), 2.91 (t, 2H), 6.30 (dd, 1H), 6.58 (d, 1H), 7.22 - 7.31 (m, 3H), 7.44 (d, 1H), 11.09 (br.s, 1H).

### 7-(2-Furanyl)-1,2,3,3a,5,9b-hexahydrocyclopenta[c]chinolin-4-on

Eine Lösung von 7-(2-Furanyl)-1,2,3,5-tetrahydrocyclopenta[c]chinolin-4-on (1.32 g, 5.3 mmol) in Methanol (300 mL) wird mit Magnesium (2.58 g, 10.6 mmol) und Essigsäure (0.06 mL) versetzt. Nach 15 h bei Raumtemp. wird eine weitere Portion Magnesium (1.29 g, 5.3 mmol) dazugegeben. Der Ansatz wird 15 h bei Raumtemp. gerührt, mit 10 proz. Salzsäure (500 mL) behandelt und mit Essigester (3 x 300 mL) extrahiert. Die vereinigten Extrakte werden getrocknet (Na₂SO₄) und i. Vak. eingeengt. Säulenchromatographie an Kieselgel mit Hexan-Essigester liefert 0.49 g Produkt.
¹H-NMR (CDCl₃): δ = 1.60 - 1.80 (m, 3H), 2.05 - 2.20 (m, 2H), 2.34 (m, 1H), 2.98 (td, 1H), 3.26 (q, 1H), 6.48 (dd, 1H), 6.64 (d, 1H), 7.04 (d, 1H), 7.22 (d, 1H), 7.32 (dd, 1H), 7.47 (d, 1H), 8.04 (br.s, 1H).

### 7-(2-Furanyl)-1,2,3,3a,5,9b-hexahydrocyclopenta[c]chinolin-4-thion

Analog zu Beispiel 4 wird 7-(2-Furanyl)-1,2,3,3a,5,9b-hexahydrocyclopenta[c]chinolin-4-on (100 mg, 0.39 mmol) mit Lawessons Reagenz (404 mg, 1.0 mmol) zu 103 mg (98%) Produkt umgesetzt.
¹H-NMR (CDCl₃): δ = 1.63 - 1.77 (m, 2H), 1.95 (m, 1H), 2.09 - 2.41 (m, 3H), 3.32 (m, 2H), 6.48 (dd, 1H), 6.65 (d, 1H), 7.16 (d, 1H), 7.25 (d, 1H), 7.40 (dd, 1H), 7.47 (d, 1H), 9.86 (br.s, 1H).

### 4-Amino-7-(2-furanyl)-2,3,3a,9b-tetrahydro-1H-cyclopenta[c]chinolin

Analog zu Beispiel 4 wird 7-(2-Furanyl)-1,2,3,3a,5,9b-hexahydrocyclopenta[c]chinolin-4-thion (95 mg, 0.35 mmol) in 7 N methanolischer Ammoniaklösung (30 mL) zu 72 mg (82%) Produkt umgesetzt.
¹H-NMR (CDCl₃) : δ = 1.58 - 1.85 (m, 3H), 1.96 (m, 1H), 2.07 - 2.33 (m, 2H), 2.91 (q, 1H), 3.32 (q, 1H), 4.61 (br., 2H), 6.43 (m, 1H), 6.61 (d, 1H), 7.15 (d, 1H), 7.31 (d, 1H), 7.35 (s, 1H), 7.43 (s, 1H);
MS (EI): m/e = 252 (M⁺)

### Beispiel 21

### 4-Amino-7-(4-morpholinyl)-2,3,3a,9b-tetrahydro-1H-cyclopenta[c]chinolin

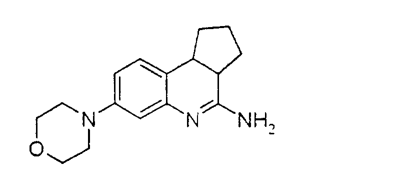

### 7-(4-Morpholinyl)-1,2,3,5-tetrahydrocyclopenta[c]chinolin-4-on

Eine Lösung von 1,1,1,3,3,3-Hexamethyldisilazan (2,6 mL, 12.5 mmol) in Toluol (100 mL) wird bei 0 °C mit 1.6 M *n*-BuLi in Hexan (7.5 mL, 12.0 mmol) versetzt und 15 min bei 0 °C gerührt. 7-Brom-1,2,3,5-tetrahydrocyclopenta[c]chinolin-4-on (1.34 g, 5.0 mmol), Morpholin (0.65 mL, 7.5 mmol), Palladiumdichlorid (44 mg, 0.25 mmol) und Tri-o-tolylphosphin (152 mg, 0.5 mmol) werden dazugegegeben. Der Ansatz wird 5 h unter Rückfluß erhitzt, mit Essigester verdünnt, mit ges. NaCl gewaschen, getrocknet (Na₂SO₄) und i. Vak. eingeengt. Reinigung des Rückstands an Kieselgel mit Dichlormethan-Ethanol liefert 1.17 g Produkt.
¹H-NMR (CDCl₃): δ = 2.19 (pent, 2H), 2.96 (t, 2H), 3.07 (t, 2H), 3.27 (dd, 4H), 3.89 (dd, 4H), 6.73 (d, 1H), 6.83 (dd. 1H), 7.39 (d, 1H), 11.04 (br.s, 1H).

### 7-(4-Morpholinyl)-1,2,3,3a,5,9b-hexahydrocyclopenta[c]chinolin-4-on

Analog zu Beispiel 7 wird 7-(4-Morpholinyl)-1,2,3,5-tetrahydrocyclopenta[c]chinolin-4-on (1.17 g, 4.3 mmol) mit Magnesium (2.16 g, 86.6 mmol) in 660 mg (56%) Produkt umgewandelt.
Fp. 210 -12 °C.

### 7-(4-Morpholinyl)-1,2,3,3a,5,9b-hexahydrocyclopenta[c]chinolin-4-thion

Analog zu Beispiel 4 wird 7-(4-Morpholinyl)-1,2,3,3a,5,9b-hexahydrocyclopenta[c]chinolin-4-on (100 mg, 0.37 mmol) mit Lawessons Reagenz (164 mg, 0.41 mmol) zu 90 mg (84%) Produkt umgesetzt.
Fp. 182 °C.

### 4-Amino-7-(4-morpholinyl)-2,3,3a,9b-tetrahydro-1H-cyclopenta[c]chinolin

Analog zu Beispiel 4 wird 7-(4-Morpholinyl)-1,2,3,3a,5,9b-hexahydrocyclopenta[c]chinolin-4-thion (80 mg, 0.28 mmol) in 7 N methanolischer Ammoniaklösung (16 mL) zu 40 mg (53%) Produkt umgesetzt.
¹H-NMR ([D₅]-Pyridin): δ = 1.43 - 1.61 (m, 2H), 1.87 (dq, 1H), 2.00 (m, 2H), 2.08 (dtd, 1H), 2.83 (q, 1H), 3.12 (dd, 4H), 3.26 (q, 1H), 3.76 (dd, 4H), 4.87 (br., 2H), 6.72 (dd, 1H), 7.12 (d, 1H), 7 17 (d, 1H)
MS (El): m/e = 271 (M⁺)

### Beispiel 22

### 4-Amino-7-(methoxycarbonylethyl)-2,3,3a,9b-tetrahydro-1H-cyclopenta[c]chinolin

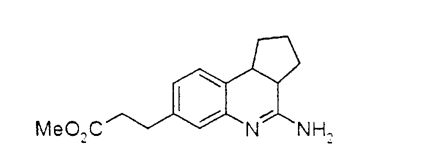

### 7-(Methoxycarbonylethenyl)-1,2,3,5-tetrahydrocyclopenta[c]chinolin-4-on

Eine Suspension von 7-Brom-1,2,3,5-tetrahydrocyclopenta[c]chinolin-4-on (528 mg, 2.0 mmol), Acrylsäure-methylester (0.36 mL, 4.0 mmol), Tetrakis(triphenylphosphin)palladium (116 mg, 0.1 mmol) und Triethylamin (0.56 mL, 4.0 mmol) in DMF (25 mL) wird 3 h bei 120 °C gerührt. Der Ansatz wird mit Essigester verdünnt, mit Wasser gewaschen, getrocknet (Na₂SO₄) und i. Vak. eingeengt. Reinigung des Rückstands an Kieselgel mit Dichlormethan-Ethanol liefert 550 mg (99%) Produkt.
¹H-NMR ([D₆]-DMSO): δ = 2.12 (pent, 2H), 2.80 (t, 2H), 3.12 (t, 2H), 3.77 (s, 3H), 6.61 (d, 1H), 7.52 (s, 1H), 7.56 (s, 2H), 7.67 (d, 1H), 11.19 (br.s, 1H).

### 7-(Methoxycarbonylethyl)-1,2,3,3a,5,9b-hexahydrocyclopenta[c]chinolin-4-on

Eine Lösung von 7-(Methoxycarbonylethenyl)-1,2,3,5-tetrahydrocyclopenta[c]chinolin-4-on (550 mg 2.0 mmol) in Methanol-THF 3:1 (130 mL) wird mit Magnesium (972 mg, 40.0 mmol) versetzt und 24 h bei Raumtemp. gerührt. Das Reaktionsgemisch wird über Glasfaser filtriert, der Filterrückstand mit Dichlormethan-Methanol gewaschen und die vereinigten Filtrate i. Vak. eingeengt. Reinigung des Rückstands an Kieselgel liefert 120 mg (22%) Produkt.
¹H-NMR (CDCl₃): δ = 1.57 - 1.77 (m, 3H), 2.02 - 2.18 (m, 2H), 2.31 (m, 1H), 2.63 (t, 2H), 2.91 (t, 2H), 2.94 (td, 1H), 3.23 (q, 1H), 3.69 (s, 3H), 6.58 (d, 1H), 6.84 (dd, 1H), 7.12 (d, 1H), 8.11 (br.s, 1H).

### 7-(Methoxycarbonylethyl)-1,2,3,3a,5,9b-hexahydrocyclopenta[c]chinolin-4-thion

Analog zu Beispiel 4 wird 7-(Methoxycarbonylethyl)-1,2,3,3a,5,9b-hexahydrocyclopenta[c]chinolin-4-on (120 mg, 0.44 mmol) mit Lawessons Reagenz (195 mg, 0.48 mmol) zu 150 mg Produkt umgesetzt.
¹H-NMR (CDCl₃): δ = 1.60 - 1.75 (m, 2H), 1.92 (m, 1H), 2.08 - 2.23 (m, 2H), 2.32 (m, 1H), 2.64 (t, 2H), 2.93 (t, 2H), 3.28 (m, 2H), 3.69 (s, 3H), 6.72 (d, 1H), 6.96 (dd, 1H), 7.18 (d, 1H), 9.80 (br.s, 1H).

### 4-Amino-7-(methoxycarbonylethyl)-2,3,3a,9b-tetrahydro-1H-cyclopenta[c]chinolin

Analog zu Beispiel 4 wird 7-(Methoxycarbonylethyl)-1,2,3,3a,5,9b-hexahydrocyclopenta[c]chinolin-4-thion (150 mg, 0.52 mmol) in 7 N methanolischer Ammoniaklösung (20 mL) zu 40 mg (28%) Produkt umgesetzt.
¹H-NMR (CDCl₃): δ = 1.61 - 1.80 (m, 3H), 1.92 (sept, 1H), 2.08 - 2.23 (m, 2H), 2.62 (t, 2H), 2.85 (q, 1H), 2.89 (t, 2H), 3.27 (q, 1H), 3.67 (s, 3H), 4.87 (br., 2H), 6.84 (dd, 1H), 6.89 (d, 1H), 7.06 (d, 1H);
MS (EI): m/e = 272 (M⁺)

### Beispiel 23

### 4-Methylamino-2,3,3a,9b-tetrahydro-1H-cyclopenta[c]chinolin

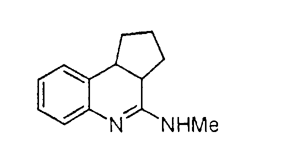

1,2,3,3a,5,9b-Hexahydrocyclopenta[c]chinolin-4-thion (90 mg, 0.44 mmol) wird in 2 M methanolischer Methylaminlösung (15 mL) 24 h bei Raumtemp. gerührt. Nach Eindampfen wird der Rückstand an Kieselgel mit Dichlormethan-Methanol gereinigt: 90 mg (99%).
¹H-NMR ([D₆]-DMSO): δ = 1.42 - 1.69 (m, 3H), 1.92 - 2.17 (m, 3H), 2.74 (m, 1H), 2.89 (s, 3H), 3.27 (m, 1H), 6.91 (t, 1H), 7.02 (d, 1H), 7.08 (t, 1H), 7.17 (d, 1H);
MS (EI) m/e = 200 (M⁺).

### Beispiel 24

### 4-Ethylamino-2,3,3a,9b-tetrahydro-1H-cyclopenta[c]chinolin

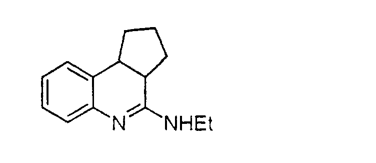

1,2,3,3a,5,9b-Hexahydrocyclopenta[c]chinolin-4-thion (90 mg, 0.44 mmol) wird in 2 M methanolischer Ethylaminlösung (15 mL) 24 h bei Raumtemp. gerührt. Nach Eindampfen wird der Rückstand an Kieselgel mit Dichlormethan-Methanol gereinigt: 60 mg (64%).
¹H-NMR ([D₆]- DMSO): δ = 1.17 (t, 3H), 1.42 - 1.67 (m, 3H), 1.92 - 2.15 (m, 3H), 2.69 (m, 1H), 3.28 (m, 1H), 3.37 (q, 2H), 6.88 (t, 1H), 6.96 (d, 1H), 7.05 (t, 1H), 7.16 (d, 1H);
MS (EI) m/e = 214 (M⁺).

### Beispiel 25

### 4-Hydroxyamino-2,3,3a,9b-tetrahydro-1H-cyclopenta[c]chinolin bzw. 1,2,3,3a,5,9b-Hexahydrocyclopenta[c]chinolin-4-oxim

Eine Suspension von 1,2,3,3a,5,9b-Hexahydrocyclopenta[c]chinolin-4-thion (50 mg, 0.25 mmol), Hydroxylammoniumsulfat (404 mg, 2.5 mmol) und Triethylamin (0.38 mL, 2.7 mmol) in Ethanol-THF 2:1 (15 mL) wird 24 h bei Raumtemp. gerührt. Das Lösungsmittel wird abdestilliert und der Rückstand an Kieselgel mit Hexan-Essigester gereinigt.
¹H-NMR ([D₆]-DMSO): δ = 1.51 - 1.77 (m, 3H), 1.87 (m, 1H), 1.95 - 2.08 (m, 2H), 2.81 (q, 1H), 3.15 (q, 1H), 6.81 (t, 1H), 7.05 (t, 1H), 7.10 (d 1H), 7.16 (d, 1H), 8.60 (br., 1H), 9.51 (br., 1H).

Analog werden erhalten:
4-Amino-7-hydroxy-2,3,3a,9b-tetrahydro-1H-cyclopenta[c]chinolin
4-Amino-7-phenoxy-2,3,3a,9b-tetrahydro-1H-cyclopenta[c]chinolin
4,7-Diamino-2,3,3a,9b-tetrahydro-1H-cyclopenta[c]chinolin
7-Acetamido-4-amino-2,3,3a,9b-tetrahydro-1H-cyclopenta[c]chinolin
4-Amino-7-(2-phenylethenyl)-2,3,3a,9b-tetrahydro-1H-cyclopenta[c]chinolin
4-Amino-7-(2-phenylethinyl)-2,3,3a,9b-tetrahydro-1H-cyclopenta[c]chinolin
7-Acetyl-4-amino-2,3,3a,9b-tetrahydro-1H-cyclopenta[c]chinolin
4-Amino-2,3,3a,9b-tetrahydro-1H-cyclopenta[c]chinolin-7-carbonsäuremethylester
4-Amino-2,3,3a,9b-tetrahydro-1H-cyclopenta[c]chinolin-7-carbonsäureethylester
4-Amino-2,3,3a,9b-tetrahydro-1H-cyclopenta[c]chinolin-7-carbonsäureamid
4-Amino-7-cyano-2,3,3a,9b-tetrahydro-1H-cyclopenta[c]chinolin
4-Amino-7-trifluormethyl-2,3,3a,9b-tetrahydro-1H-cyclopenta[c]chinolin
4-Amino-7-chlor-2,3,3a,9b-tetrahydro-1H-cyclopenta[c]chinolin
4-Amino-7-brom-2,3,3a,9b-tetrahydro-1H-cyclopenta[c]chinolin
4-Amino-7-methylthio-2,3,3a,9b-tetrahydro-1H-cyclopenta[c]chinolin
4-Amino-7-(4-methy)-1-piperazinyl)-2,3,3a,9b-tetrahydro-1Hcyclopenta[c]chinolin
4-Amino-6-methoxy-2,3,3a,9b-tetrahydro-1H-cyclopenta[c]chinolin
4-Amino-8-methoxy-2,3,3a,9b-tetrahydro-1H-cyclopenta[c]chinolin
4-Amino-2-thia-2,3,3a,9b-tetrahydro-1H-cyclopenta[c]chinolin
4-Amino-2-methyl-2-aza-2,3,3a,9b-tetrahydro-1H-cyclopenta[c]chinolin
4-n-Propylamino-2,3,3a,9b-tetrahydro-1H-cyclopenta[c]chinolin
4-Amino-6,7-difluor-2,3,3a,9b-tetrahydro-1H-cyclopenta[c]chinolin
4-Amino-8-chlor-6,7-difluor-2,3,3a,9b-tetrahydro-1H-cyclopenta[c]chinolin

## Patentansprüche

1. Verbindungen der Formel I, deren tautomere und isomere Formen und physiologisch verträglichen Salze 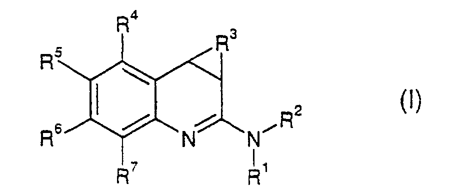 worin
R¹ und R² unabhängig voneinander bedeuten
a) Wasserstoff,
b) C₁₋₆-Alkyl,
c) OR⁸,
d) NR⁸R⁹,
e) CN,
f) C₁₋₆-Alkanoyl, gegebenenfalls mit Halogen und C₁₋₄-Alkyl substituierte Benzolsulfonsäure oder C₁₋₄-Alkansulfonsäure,
g) CO₂R¹⁰,
h) CONR⁸R⁹,
i) CSNR⁸R⁹,
R3 bedeutet
einen gesättigten oder ungesättigten C₁₋₅-Alkylenrest, der 1- bis 4-fach mit OR¹¹, NR¹²R¹³ oder C₁₋₄-Alkyl substituiert sein kann oder bei dem 1 oder 2 CH₂-Gruppen durch O, S(O)ₙ, NR¹⁴ , =N- oder Carbonyl ersetzt sein können und der mit einer Methano-, Ethano- oder Propano-Gruppe überbrückt sein kann,
R⁴, R⁵, R⁶ und R⁷ unabhängig voneinander bedeuten
a) Wasserstoff,
b) Halogen,
c) S(O)ₙR¹⁶,
d) OR¹⁶,
e) COOR¹⁶,
f) COR¹⁶,
g) CONR¹⁶R¹⁷,
h) CSNR¹⁶R¹⁷,
i) C(NR¹⁸)NR¹⁶R¹⁷,
j) NR¹⁶R¹⁹,
k) C₁₋₆-Alkyl, das gegebenenfalls substituiert ist mit Halogen, OR¹⁶, SR¹⁶, COOR ¹⁶, Phenyl, 5-6-gliedrigem Heteroaryl mit 1-4 N-, S- oder O-Atomen oder C₃₋₇-Cycloalkyl,
l) C₃₋₇-Cycloalkyl,
m) C₂₋₆-Alkenyl, gegebenenfalls substituiert mit Phenyl oder Halogen,
n) C₂₋₆-Alkinyl, gegebenenfalls substituiert mit Phenyl oder Halogen,
o) C₆₋₁₀-Aryl, das gegebenenfalls substituiert ist mit Halogen, CN, C₁₋₄-Alkyl, SR¹⁶ oder OR¹⁶,
p) 5-6-gliedriges Hetaryl mit 1 bis 4 N-, O- oder S-Atomen, das einen ankondensierten Benzolring enhalten und substituiert sein kann mit Halogen, NO₂, Cyano, -OR¹⁶, SR¹⁶, C₁₋₄-Alkyl, CF₃ oder NR¹⁶R¹⁷
q) CN,
r) N02,
s) CF3,
t) OCF₃,
R⁸, R⁹, R¹⁰, R¹¹, R¹⁶, R¹⁸, R²¹ und R²² unabhängig voneinander bedeuten
a) Wasserstoff,
b) C₁₋₆-Alkyl,
c) C₆₋₁₀-Aryl, das gegebenenfalls substituiert ist mit Halogen oder C₁₋₄-Alkyl
R²⁰ bedeutet
a)C₁₋₆-Alkyl,
b) C₆₋₁₀-Aryl bedeutet, das gegebenenfalls substituiert ist mit Halogen oder C₁₋₄-Alkyl,
R¹², R¹³, R¹⁴ und R¹⁹ unabhängig voneinander bedeuten
a) Wasserstoff,
b) C₁₋₆-Alkyl,
b) COR²⁰,
c) CO₂R²¹,
d) CONR²¹R²²,
e) CSNR²¹R²²,
R¹⁷ bedeutet
a) Wasserstoff,
b) C₁₋₆-Alkyl, gegebenenfalls substituiert mit Halogenen, Amino-, Hydroxy- oder Sulfhydrylgruppen,
c) C₆₋₁₀-Aryl,
n bedeutet 0,1,2 und
R¹⁶, R¹⁹ gemeinsam mit dem Stickstoffatom einen gesättigten 5-, 6- oder 7-gliedrigen Ring bilden, der ein weiteres Stickstoff-, Sauerstoff- oder Schwefelatom enthalten und substituiert sein kann mit C₁₋₄-Alkyl oder mit einem gegebenenfalls halogenierten Phenyl-, Benzyl- oder Benzoyl-Rest, wobei 2H,2-Methyl-pyrazolo[3,4-c]-chinalin-4-N,N-diethylamin ausgeschlossen ist.

2. Verbindungen nach Anspruch 1, worin R¹ und R² unabhängig voneinander Wasserstoff, C₁₋₆-Alkyl oder Hydroxy bedeuten.

3. Verbindungen nach Anspruch 1 und 2, worin R³ Alkylen mit 1 bis 5 Kohlenstoffatomen bedeutet, bei dem eine CH₂-Gruppe durch Sauerstoff oder Schwefel ersetzt sein kann.

4. Verbindungen nach Anspruch 1, 2 und 3, worin R⁴, R⁵, R⁶ und R⁷ unabhängig voneinander
a) Wasserstoff,
b) Halogen,
c) SR¹⁶,
d) OR¹⁶,
e) COOR¹⁶,
f) COR¹⁶,
g) CONR¹⁶R¹⁷,
h) NR¹⁶R¹⁹,
i) C₁₋₆-Alkyl, das gegebenenfalls substituiert ist mit Halogen, COOR¹⁶ oder Phenyl,
j) C₂₋₆-Alkenyl, gegebenenfalls substituiert mit Phenyl oder Halogen,
k) C₂₋₆-Alkinyl, gegebenenfalls substituiert mit Phenyl oder Halogen,
l) Phenyl, das gegebenenfalls substituiert ist mit Halogen, CN, C₁₋₄-Alkyl, SR¹⁶ oder OR¹⁶,
m) 5-6-gliedriges Heteroaryl mit 1 bis 4 N-, O- oder S-Atomen, das mit Halogen, NO₂, Cyano, C₁₋₄-Alkyl oder CF₃ substituiert sein kann,
n) CN,
o) NO₂,
p) CF₃,
q) OCF₃ bedeuten.

5. 4-Amino-2,3,3a,9b-tetrahydro-1H-cyclopenta[c]chinolin
6-Amino-6a,7,8,9,10,10a-hexahydrophenanthridin
6-Amino--2,3,4,5,5a,11b-hexahydro-1H-cyclohepta[c]chinolin
nach Anspruch 1-4.

6. Arzneimittel enthaltend eine Verbindung nach Anspruch 1 bis 5.

7. Verfahren zur Herstellung von Verbindungen nach Anspruch 1 dadurch, daß man eine Verbindung der Formel (II) oder deren Salz 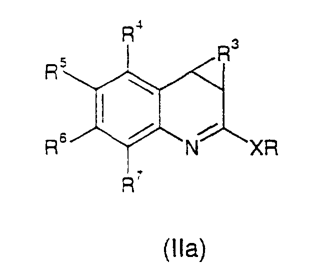 oder 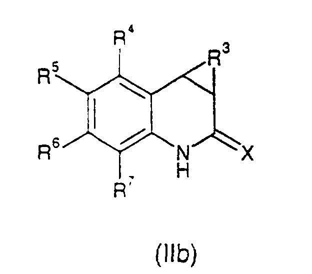 worin R³ bis R⁷ die obige Bedeutung haben, R Methyl oder Ethyl und X Sauerstoff oder Schwefel ist, mit Ammoniak, primären oder sekundären Aminen, Hydroxylamin und seinen Derivaten oder Hydrazin und seinen Derivaten umsetzt und gewünschtenfalls anschließend die Isomeren trennt oder die Salze bildet.

8. Verwendung der Verbindungen der Formel I nach Anspruch 1 zur Herstellung eines Arzneimittels zur Behandlung von Erkrankungen, die durch NOS-Synthasen ausgelöst werden.

## Claims

1. Compounds of Formula I, their tautomeric and isomeric forms and their physiologically compatible salts, 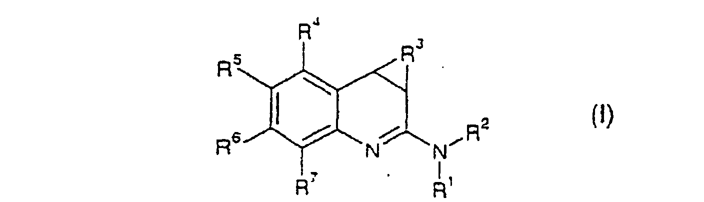 in which
R¹ and R², independently of one another, mean:
a) hydrogen,
b) C₁₋₆ alkyl,
c) OR⁸,
d) NR⁸R⁹,
e) CN,
f) C₁₋₆ alkanoyl, optionally halogen-substituted and C₁₋₄ alkyl-substituted benzenesulfonic acid or C₁₋₄ alkanesulfonic acid,
g) CO₂R¹⁰,
h) CONR⁸R⁹,
i) CSNR⁸R⁹,
R³ means:
a saturated or unsaturated C₁₋₅ alkylene radical, which can be substituted in 1 to 4 places with OR¹¹, NR¹²R¹³ or C₁-₄ alkyl or in which 1 or 2 CH₂ groups can be replaced by O, S(O)ₙ, NR¹⁴, =N- or carbonyl, and which can be bridged with a methane, ethano or propane group,
R⁴, R⁵, R⁶ and R⁷, independently of one another, mean:
a) hydrogen,
b) halogen,
c) S(O)ₙR¹⁶,
d) OR¹⁶,
e) COOR¹⁶,
f) COR¹⁶,
g) CONR¹⁶R¹⁷,
h) CSNR¹⁶R¹⁷,
i) C(NR¹⁸)NR¹⁶R¹⁷,
j) NR¹⁶R¹⁹,
k) C₁₋₆ alkyl, which optionally is substituted with halogen, OR¹⁶, SR¹⁶, COOR¹⁶, phenyl, 5- to 6-membered heteroaryl with 1-4 N, S or O atoms or C₃₋₇ cycloalkyl,
l) C₃₋₇ cycloalkyl,
m) C₂₋₆ alkenyl, optionally substituted with phenyl or halogen,
n) C₂₋₆ alkynyl, optionally substituted with phenyl or halogen,
o) C₆₋₁₀ aryl, which optionally is substituted with halogen, CN, C₁₋₄ alkyl, SR¹⁶ or OR¹⁶,
p) 5- to 6-membered hetaryl with 1 to 4 N, O or S atoms, which can contain a fused-on benzene ring and can be substituted with halogen, NO₂, cyano, -OR¹⁶, SR¹⁶, C₁₋₄ alkyl, CF₃ or NR¹⁶R¹⁷,
q) CN,
r) NO₂,
s) CF₃,
t) OCF₃,
R⁸, R⁹, R¹⁰, R¹¹, R¹⁶, R¹⁸, R²¹ and R²², independently of one another, mean:
a) hydrogen,
b) C₁₋₆ alkyl,
c) C₆₋₁₀ aryl, which optionally is substituted with halogen or C₁₋₄ alkyl,
R²⁰ means
a) C₁₋₆ alkyl,
b) C₆₋₁₀ aryl, which optionally is substituted with halogen or C₁₋₄ alkyl,
R¹², R¹³, R¹⁴ and R¹⁹, independently of one another, mean:
a) hydrogen,
b) C₁₋₆ alkyl,
c) COR²⁰,
d) CO₂R²¹,
e) CONR²¹R²²,
f) CSNR²¹R²²,
R¹⁷ means
a) hydrogen,
b) C₁₋₆ alkyl, optionally substituted with halogens, amino, hydroxy or sulfhydryl groups,
c) C₆₋₁₀ aryl,
n means 0, 1, 2; and
R¹⁶ and R¹⁹ together with the nitrogen atom form a saturated 5-, 6- or 7-membered ring, which can contain another nitrogen, oxygen, or sulfur atom and can be substituted with C₁₋₄ alkyl or with an optionally halogenated phenyl, benzyl or benzoyl radical,
with the exception of 2H,2-methylpyrazolo[3,4-c]quinoline-4-N,N-diethylamine.

2. Compounds according to Claim 1, in which R¹ and R², independently of one another, mean hydrogen, C₁₋₆ alkyl or hydroxy.

3. Compounds according to Claim 1 and 2, in which R³ means alkylene with 1 to 5 carbon atoms, in which a CH₂ group can be replaced by oxygen or sulfur.

4. Compounds according to claim 1, 2 and 3, in which R⁴, R⁵, R⁶ and R⁷, independently of one another, mean
a) hydrogen
b) halogen
c) SR¹⁶,
d) OR¹⁶,
e) COOR¹⁶
f) COR¹⁶,
g) CONR¹⁶R¹⁷,
h) NR¹⁶R¹⁹,
i) C₁₋₆ alkyl, which optionally is substituted with halogen, COOR¹⁶ or phenyl,
j) C₂₋₆ alkenyl, optionally substituted with phenyl or halogen,
k) C₂₋₆ alkynyl, optionally substituted with phenyl or halogen,
l) phenyl, which optionally is substituted with halogen, CN, C₁₋₄ alkyl, SR¹⁶ or OR¹⁶,
m) 5-6-membered hetaryl with 1 to 4 N, O or S atoms, which can be substituted with halogen, NO₂, cyano, C₁₋₄ alkyl or CF₃,
n) CN,
o) NO₂,
p) CF₃,
q) OCF₃.

5. 4-Amino-2,3,3a,9b-tetrahydro-1H-cyclo-penta[c]quinoline
6-amino-6a,7,8,9,10,10a-hexahydrophenanthridine
6-amino-2,3,4,5,5a,11b-hexahydro-1H-cyclo-hepta[c]quinoline
according to Claim 1-4.

6. Pharmaceutical compositions comprising a compound according to Claim 1 to 5.

7. Process for the production of compounds according to Claim 1 by reacting a compound of Formula (II) or its salt 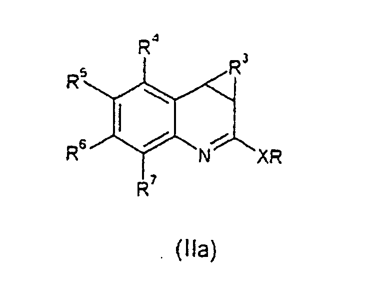 or 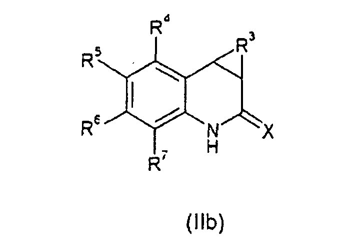 in which R³ to R⁷ have the above meaning, R is methyl or ethyl and X is oxygen or sulfur, with ammonia, primary or secondary amines, hydroxylamine and its derivatives or hydrazine and its derivatives, and optionally then separating the isomers or forming the salts.

8. Use of the compounds of Formula I according to Claim 1 for the production of a pharmaceutical composition for treating diseases caused by NO-synthases.

## Revendications

1. Composés de formule I, leurs formes tautomères et isomères et sels physiologiquement acceptables 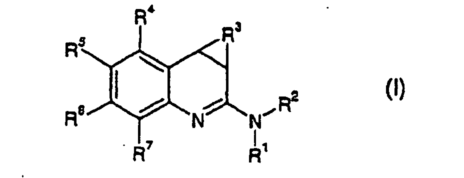 dans laquelle
R¹ et R² signifient, indépendamment l'un de l'autre
a) hydrogène,
b) alkyle en C₁ à C₆,
c) OR⁸,
d) NR⁸R⁹,
e) CN,
f) alcanoyle en C₁ à C₆, acide benzènesulfonique ou acide (alcane en C₁ à C₄) sulfonique éventuellement substitué par halogène et alkyle en C₁ à C₄,
g) CO₂R¹⁰,
h) CONR⁸R⁹,
i) CSNR⁸R⁹,
R³ signifie
un radical alkylène en C₁ à C₅ saturé ou insaturé, qui peut être monosubstitué à tétrasubstitué par OR¹¹, NR¹²R¹³ ou alkyle en C₁ à C₄ ou dans lequel un ou deux groupes CH₂ peuvent être remplacés par O, S(O)ₙ, NR¹⁴, =N- ou carbonyle et qui peut être dans un pont avec un groupe méthano, éthano ou propano,
R⁴, R⁵, R⁶ et R⁷ signifient indépendamment l'un de l'autre
a) hydrogène,
b) halogène,
c) S (O)ₙR¹⁶,
d) OR¹⁶,
e) COOR¹⁶,
f) COR¹⁶,
g) CONR¹⁶R¹⁷,
h) CSNR¹⁶R¹⁷,
i) C(NR¹⁸)NR¹⁶R¹⁷,
j) NR¹⁶R¹⁹,
k) alkyle en C₁ à C₆, qui est éventuellement substitué par halogène, OR¹⁶ SR¹⁶, COOR¹⁶, phényle, hétéroaryle de 5 à 6 chaînons, avec 1 à 4 atomes de N, S ou O ou cycloalkyle en C₃ à C₇,
l) cycloalkyle en C₃ à C₇,
m) alcényle en C₂ à C₆, éventuellement substitué par phényle ou halogène,
n) alcynyle en C₂ à C₆, éventuellement substitué par phényle ou halogène,
o) aryle en C₆ à C₁₀, éventuellement substitué par halogène, CN, alkyle en C₁ à C₄, SR¹⁶ ou OR¹⁶,
p) hétéroaryle de 5 à 6 chaînons, comprenant 1 à 4 atomes de N, O ou S, qui peut contenir un cycle benzène condensé et qui peut être substitué par halogène, NO₂, cyano, -OR¹⁶, SR¹⁶, alkyle en C₁ à C₄, CF₃ ou NR¹⁶R¹⁷,
q) CN,
r) NO₂,
s) CF₃,
t) OCF₃,
R⁸, R⁹, R¹⁰, R¹¹, R¹⁶, R¹⁸, R²¹ et R²² signifient, indépendamment l'un de l'autre
a) hydrogène,
b) alkyle en C₁ à C₆,
c) aryle en C₆ à C₁₀, qui est éventuellement substitué par halogène ou alkyle en C₁ à C₄,
R²⁰ signifie
a) alkyle en C₁ à C₆,
b) aryle en C₆ à C₁₀, qui est éventuellement substitué par halogène ou alkyle en C₁ à C₄,
R¹², R¹³, R¹⁴ et R¹⁹ signifient, indépendamment l'un de l'autre
a) hydrogène,
b) alkyle en C₁ à C₆,
b) COR²⁰,
c) CO₂R²¹,
d) CONR²¹R²²,
e) CSNR²¹R²²,
R¹⁷ signifie
a) hydrogène,
b) alkyle en C₁ à C₆, éventuellement substitué par halogène, des groupes amino, hydroxy ou sulfhydryle,
c) aryle en C₆ à C₁₀,
n signifie 0, 1, 2 et
R¹⁶, R¹⁹ forment avec l'atome d'azote un cycle saturé de 5, 6 ou 7 chaînons, qui peut contenir un autre atome d'azote, d'oxygène ou de soufre et qui peut être substitué par alkyle en C₁ à C₄ ou par un radical phényle, benzyle ou benzoyle éventuellement halogéné,
la 2H,2-méthyl-pyrazolo[3,4-c]-quinoléine-4-N,N-diéthylamine étant exclue.

2. Composés selon la revendication 1, dans lesquels R¹ et R² signifient, indépendamment l'un de l'autre, hydrogène, alkyle en C₁ à C₆ ou hydroxy.

3. Composés selon la revendication 1 et 2, dans lesquels R³ signifie alkylène comprenant 1 à 5 atomes de carbone, dans lequel un groupe CH₂ peut être remplacé par oxygène ou soufre.

4. Composés selon la revendication 1, 2 et 3, dans lesquels R⁴, R⁵, R⁶ et R⁷ signifient, indépendamment l'un de l'autre
a) hydrogène,
b) halogène,
c) SR¹⁶,
d) OR¹⁶,
e) COOR¹⁶,
f) COR¹⁶,
g) CONR¹⁶R¹⁷,
h) NR¹⁶R¹⁹,
i) alkyle en C₁ à C₆, qui est éventuellement substitué par halogène, COOR¹⁶ ou phényle,
j) alcényle en C₂ à C₆, éventuellement substitué par phényle ou halogène,
k) alcynyle en C₂ à C₆, éventuellement substitué par phényle ou halogène,
l) phényle, éventuellement substitué par halogène, CN, alkyle en C₁ à C₄, SR¹⁶ ou OR¹⁶,
m) hétéroaryle de 5 à 6 chaînons, comprenant 1 à 4 atomes de N, O ou S, qui peut être substitué par halogène, NO₂, cyano, alkyle en C₁ à C₄ ou CF₃,
n) CN,
o) NO₂,
p) CF₃,
q) OCF₃.

5. 4-amino-2,3,3a,9b-tétrahydro-1H-cyclopenta[c]-quinoléine
6-amino-6a,7,8,9,10,10a-hexahydrophénanthridine
6-amino-2,3,4,5,5a,11b-hexahydro-1H-cyclohepta[c]-quinoléine selon la revendication 1 à 4.

6. Médicament contenant un composé selon la revendication 1 à 5.

7. Procédé pour la préparation de composés selon la revendication 1, en ce qu'on transforme un composé de formule (II) ou son sel 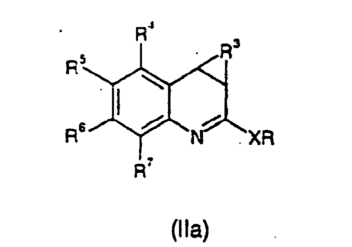 ou 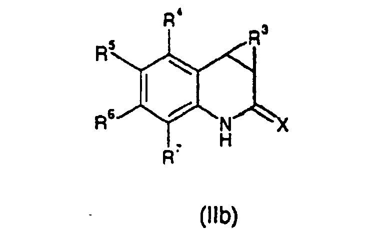 dans lesquelles R³ à R⁷ ont la signification susmentionnée, R représente méthyle ou éthyle et X représente oxygène ou soufre, avec de l'ammoniaque, des amines primaires ou secondaires, de l'hydroxylamine et ses dérivés ou de l'hydrazine et ses dérivés et qu'on sépare ensuite, si souhaité, les isomères ou qu'on forme les sels.

8. Utilisation des composés de formule I selon la revendication 1 pour la préparation d'un médicament pour le traitement de maladies déclenchées par les NOS-synthases.
